# EUROPÄISCHE PATENTSCHRIFT

(11) **EP 2 086 484 B1**
(45) Veröffentlichungstag und Bekanntmachung des Hinweises auf die Patenterteilung: **07.05.2014**
(21) Anmeldenummer: 07820691.9
(22) Anmeldetag: 28.09.2007
(51) Int. Cl.: A61F 13/00

(54) **WUNDPFLEGEARTIKEL ZUR EXTRAKTION UND KONTROLLE VON WUNDFLÜSSIGKEITEN**
WOUND CARE ARTICLE FOR EXTRACTION AND CONTROL OF WOUND FLUIDS
ARTICLE DE TRAITEMENT DE BLESSURES EN VUE DE L'EXTRACTION ET DU CONTRÔLE DE FLUIDES DE BLESSURES

(30) Priorität: 02.10.2006 DE 102006047041
(43) Veröffentlichungstag der Anmeldung: 12.08.2009
(60) Teilanmeldung: 12185483.0
(73) Patentinhaber: Riesinger, Birgit, 48149 Münster (DE)
(72) Erfinder: Riesinger, Birgit, 48149 Münster (DE)
(74) Vertreter: Michalski Hüttermann & Partner Patentanwälte
(86) Internationale Anmeldenummer: PCT/EP2007/060304
(87) Internationale Veröffentlichungsnummer: WO 2008/040681

(56) Entgegenhaltungen:
- EP-B- 0 762 860
- WO-A-83/02054
- GB-A- 2 272 645
- US-A- 3 871 376
- US-A- 3 872 862
- US-A- 5 476 664

## Beschreibung

Die vorliegende Annmeldung beansprucht die Priorität der DE 102006047041, auf deren Offenbarungsgehalt hier vollumfänglich Bezug genommen wird. Der Offenbarungsgehalt der DE 102006047041 ist im Anhang dieser Anmeldung beigefügt.

Die Erfindung betrifft einen kissenförmigen Wundpflegeartikel zur Extraktion und Kontrolle von Wundflüssigkeiten gemäß dem Oberbegriff des Anspruchs 1.

Ein solcher Wundpflegeartikel eignet sich insbesondere zur Aufnahme von Exsudat aus chronischen Wunden, wie z.B. bei Diabetes, Ulcus cruris und ähnlichen Erkrankungen auftreten.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilüngsverlaufs entsprechend der verschiedenen Phasen der Wundheilung, so spricht manvon einer chronischen Wunde. Dabei betrachtet man jedoch bereits länger als drei Tageandauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvo rangesprochenen Matrix-Metalloproteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer aktiven Wundregression beiträgt. Das pathologische Exsudat ist hinsichtlich des Gehalts seiner Komponenten aus dem der Wundprogressionförderlichen Gleichgewicht geraten. Daraus ergeben sich verschiedene Komplikationen,die zur weiteren Verschlechterung und Chronifizierung der Wunde beitragen.

Ein Wundpflegeartikel der eingangs genannten Art dient insbesondere dazu, besagtes chronisches Exsudat zu absorbieren und so die Wundheilung zu fördern. Ein solcher Wundpflegeartikel ist aus DE 100 59 439 der Anmelderin bekannt. Bei dem bekannten Wundpflegeartikel handelt sich um ein Wundpflegeartikel, deren zusätzliche, innere Umhüllung aus Baumwolle die Funktion hat, den direkten Kontakt zwischen den Schleimhautzellen an der Wunde oder Körperhöhle des Patienten und der saugfähigen Matte zu erschweren. Die Wirkung des auf die Wunde oder Körperhöhle des Patienten aufgelegten Wundpflegeartikels - insbesondere die auf die Wunde ausgeübte Saugwirkung - ist gleich, egal ob sich um seine "linke" oder "rechte" Seite handelt.

WO 83/02054 A (KAMME, Carl Gustaf) 23. Juni 1983 (1983-06-23) beschreibt einen Wundpflegeartikel, der aus einem flüssigkeitsabsorbierenden Körper, einer Ummantelung und einer möglichen wundschützenden Schicht besteht.

GB 2 272 645 A (Johnson & Johnson Medical Inc. USA) 25. Mai 1994 (1994-05 -25) beschreibt eine mehrschichtige Wundauflage zur Behandlung von geschädigter, verbrannter, eitriger oder anderweitig traumatisierter Haut.

US 3 872 862 A (HUME, Michael) 25. März 1975 (1975-03-25) beschreibt eine medizinische Wundauflage zur Behandlung von Geschwüren, bedingt durch chronische Insuffizienz der Venenklappen.

US 3 871 376 A (KOZAK, Theodore F.)18. März 1975 (1975-03-18) beschreibt eine Kombination eines absorbierenden Verbandmaterials und einer flexibel, kühlenden Auflage, das aus einer aufgebrachten Kühlauflage über einer Wundauflage sowie aus einem isolierenden Material besteht, dass besagte Kühl- und Wundauflage umschließt.

Aufgabe der Erfindung ist, einen gattungsgemäßen Wundpflegeartikel bereitzustellen, der dem behandelnden Personal eine größere Flexibilität bei der Wundversorgung ermöglicht. Eine weitere Aufgabe ist es, einen gattungsgemäßen Wundpflegeartikel bereitzustellen, der dem behandelnden Personal die Möglichkeit gibt, entsprechend der Erfordernisse der zu behandelnden Wunde unterschiedlich zu reagieren. Weitere Aufgabe ist es, einen gattungsgemäßen Wundpflegeartikel bereitzustellen, der sich mit seiner einer Flachseite zur Auflage auf schwach bis mäßig und mit seiner anderen Flachseite auf eher stark exsudierendc bzw. sezernierende Wunden eignet.

Diese Aufgaben werden durch einen Wundpflegeartikel mit den Merkmalen des vorgelegten Hauptanspruchs gelöst. Die Unteransprüche geben bevorzugte Ausführungsformen an. Dabei ist zu beachten, dass die genannten Bereichsangaben durchweg einschließlich der jeweiligen Grenzwerte zu verstehen sind.

Demnach ist ein Wundpflegeartikel zur Extraktion und Kontrolle von Wundflüssigkeiten vorgesehen, aufweisend wenigstens einen ersten flüssigkeitsabsorbierenden Körper, der von einer flüssigkeitsdurchlässigen, ersten Hülle umgeben ist, und eine flüssigkeitsdurchlässige, aus zwei Hüllenwänden bestehende, zweite Hülle. Der Wundpflegeartikel ist dadurch gekenntzeichnet, dass die erste Hülle an wenigstens einer ihrer Flachseiten von wenigstens einer flüssigkeitsabsorbierenden Materiallage be- oder unterlegt ist, die zwischen der ersten Hülle (2) und einer der Hüllenwände der zweiten Hülle angeordnet ist. Dabei liegt der erste flüssigkeitsabsorbierende Körper (1; 10) in Form einer vliesartigen Matte vor und weist eine superabsorbierende Substanz auf. Die flüssigkeitsabsorbierende Materiallage enthält Methylcellulose.

Die erste Hülle kann von der zweiten Hülle umgeben sein. Bevorzugt ist in diesem Zusammenhang vorgesehen, dass der erste flüssigkeitsabsorbierende Körper von einer ebenfalls flüssigkeitsdurchlässigen, aus zwei Hüllenwänden bestehenden inneren Hülle umgeben ist. In dieser "Hülle in Hülle"-Ausführungsform ist also die flüssigkeitsabsorbierende Materiallage nur von der zweiten, äußeren Hülle umgeben, während der erste flüssigkeitsabsorbierende Körper von der äußeren und der ersten, d. h. inneren Hülle umgeben ist.

Der Begriff "Wundpflegeartikel" soll im Folgenden insbesondere eine Wundauflage, bevorzugt eine flächige Wundauflage oder ein Wundpflegetuch bezeichnen. Besagte Wundauflage kann dabei sowohl absorbierend als auch nicht oder nur unwesentlich absorbierend ausgestaltet sein. Insbesondere kann der Begriff "Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte mithilfe einer Wickel in der gegebenen Anordnung auf der zu behandelnden Wunde angeordnet sind.

Bei einer anderen Ausführungsform können die erste Hülle und die zweite Hülle mit darin untergebrachten erstem Körper und der Materiallage ein Verbandheft bilden, indem die beiden Hüllen über wenigstens eine Faltlinie oder wenigstens eine Längs- oder Punktnaht zusammenklappbar sind. Vorzugsweise geht die erste Hülle in einem Materialstück in die zweite Hülle über.

Bei der flüssigkeitsabsorbierenden Materiallage handelt es sich bevorzugt um eine Schaum-stoff-Matte, die auch Cellulosefasern oder -partikeln enthalten werden kann. Die Materiallage kann hydrofaser- oder/und alginathaltig sein. Die Materiallage kann aber auch aus anderen Materialien bestehen, solange diese in der Lage sind, Flüssigkeiten zu absorbieren.

Bei dem erwähnten Schaumstoff handelt es sich bevorzugt um Polyurethane, beispielsweise "cross-linked" Schäume.

Dabei ist insbesondere vorgesehen, dass der erste flüssigkeitsabsorbierende Körper eine höhere Aufnahmekapazität und ein höheres Saugvermögen aufweist als der zweite flüssigkeitsabsorbierende Körper, d. h. die vorgenannte Material-Lage.

Unter dem Begriff "kissenförmig" soll im folgenden ein im wesentlichen flächiger Körper verstanden werden, der im Wesentlichen aus zwei seitlichen Wänden besteht, die die äußere Hülle ausmachen, wobei die seitlichen Wände an ihren Rändern über Kleb-, Druck-, Schweiß oder Thermoverbindungen mit einander verbunden sind und Nähte oder Säume ausbilden. Da die äußere Hülle eine flüssigkeitsabsorbierende Materiallage Matte umgibt, ergibt sich bei dieser Konfiguration eine abgeflachte Kissenform, die mit zunehmender Flüssigkeitsaufnahme einen immer stärker gewölbten Querschnitt, insbesondere auf der Seite des Wundpflegeartikels, auf der sich der flüssigkeitsabsorbierende Körper befindet.

Unter dem Begriff "Extraktion und Kontrolle von Wundflüssigkeiten" soll im Folgenden verstanden werden, dass der erfindungsgemäße Wundpflegeartikel Wundflüssigkeiten aktiv aufnimmt und festhält. Bei diesen Wundflüssigkeiten handelt es sich insbesondere um an sich bekannte pathologische, grundsätzlich länger als 48-72 Stunden ausfließende Exsudate. Dieser Begriff impliziert überdies, dass die beiden Absorptionskörper für die Verwendung als Wundpflegeartikel vorgeschen sind.

Der erste flüssigkeitsabsorbierende Körper und die Materiallage können flächenmäßig gleich sein, bevorzugt wird jedoch ein Aufbau des Wundpflegeartikels, bei dem der erste flüssigkeitsabsorbierendc Körper flächenmäßig kleiner als die Materiallage ist. Dabei ergeben sich unterschiedliche Aufbaumöglichkeiten:
- Die Hüllenwand der zweiten Hülle kann zugleich eine Hüllenwand der ersten Hülle bilden, wobei die Hüllenwand der ersten Hülle über eine umlaufende Naht mit der Hüllenwand der zweiten Hülle verbunden sein kann;
- Die aus zwei Hüllenwänden bestehende erste Hülle kann über eine umlaufende Naht mit der Hüllenwand der zweiten Hülle verbunden sein,
wobei in beiden Fällen ist die Bedingung erfüllt, dass die erste Hülle flächenmäßig kleiner als die zweite Hülle ist.

Weiterhin kann bei dem bereits beschriebenen Verbandheft der erste flüssigkeitsabsorbierende Körper ebenso flächenmäßig kleiner als die Materiallage sein.

Bei dem ersten flüssigkeitsabsorbierenden Körper handelt es sich um eine vliesartige Matte. Die letztere kann cellulose-, insbesondere carboxymethylcellulosehaltig oder alginathaltig sein. Die Matte kann auch gewebeartig sein.

Alginate werden aus den Braunalgen gewonnen und zu einem faserigen Vlies verwoben Chemisch handelt es sich um Polysaccharide, und zwar Calciumund/oder Natrimsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca<2+> Ionen werden gegen die Na<+> Ionen aus dem Exsudat ausgetauscht, bis der Sättigungsgrad an Na-Ionen im Alginat erreicht ist. Dabei kommt es zu einem Aufquellen des Wundpflegeartikels und zur Umwandlung der Alginatfaser in einen Gelkörper durch Aufquellen der Fasern.

Carboxymethylcellulose liegt insbesondere in Form von Natriumcarboxymethylcellulose vor und ist u. a. unter dem Namen "Hydrofaser" im Handel. In Hygieneund Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fliesst. Auf diese Weise kann eine Wundrandmazeration effektiv verhindert werden.

Die insbesondere aus Natriumcarboxymethylcellulose hergestellten, mattenartigen Absorptionskörper sind in ihren Materialeigenschaften, den Wirkungsweisen sowie den Indikationsspektren den Alginatprodukten sehr ähnlich. Sie werden auch in trockenem Zustand auf die Wunde aufgebracht, saugen relativ stark und schnell Wundexsudat auf und werden dabei in ein durchsichtiges, trübes Gel umgewandelt. Die Sckretaufnahmekapazität ist hoch und erfolgt in vertikaler Richtung. Somit entsteht nur im Bereich der feuchten Wundfläche das gewünschte Gel. Wundrand und Wundumgebung bleiben trocken, es entsteht keine Mazeration.

Vorzugsweise weist der erste flüssigkeitsabsorbierende und/oder die Materiallage mindestens eine superabsorbierende Substanz auf. Hierbei handelt es sich bevorzugt um Superabsorbierende Polymere (SAP) die ggf. in einer Cellulosefasern enthaltenden Matrix angeordnet sind. Diese bevorzugt verwendeten Superabsorber sind ein Grund dafür, dass der erste flüssigkeitsabsorbierende Körper eine höhere Aufnahmekapazität und/oder Saugkraft aufweist als die Natriumcarboxymethylcellulose-, Algniat- oder Schaumstoffmatte.

Superabsorbierende Polymere (SAP) werden sind Kunststoffe die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvemetzer (Core-Cross-Linker, CXL) der Monomerlösung zugesetzt, der die gebildeten langkettigen Polymermoleküle stellenweise untereinander durch chemische Brücken verbindet (sie "vemetzt"). Durch diese Brücken wird das Polymer wasserunlöslich. Beim Eindringen von Wasser oder wässrigen Salzlösungen in die Polymerpartikeln quellen sie auf und straffen auf molekularer Ebene dieses Netzwerk so dass das Wasser ohne Hilfe nicht mehr entweichen kann.

Die superabsorbierenden Polymere können in dem erfindungsgemäßen Wundpflegeartikel in Form eines Granulats, eines Pulvers, einer Schüttung, eines Presslings, und/oder eines Schaums vorliegen.

Die Superabsorber-Teilchen können in Pulver- oder Granulatform von einer Partikelgröße zwischen 100 und etwa 1000 µm vorliegen. Das Gewichtsverhältnis zwischen Superabsorber und dem flüssigkeitsabsorbierenden Körper liegt im Bereich 0,01 bis etwa 0,5.

Die den ersten flüssigkeitsabsorbierenden Körper bildende, cellulosehaltige Matte kann vom Typ Airlaid sein.

Ferner ist es möglich, den ersten flüssigkeitsabsorbierenden Körper in Form einer Schüttung eines Presslings und/oder in Form von Schnipseln herzustellen. Die Schüttung oder der Pressling kann dabei entweder aus mehreren aus der Matte hergestellten Schnipseln, d.h. Schnittproduktcn, oder ausschließlich aus pulver-, granulat- oder faserförmigen Superabsorberteilchen besteht. Die Schnipsel selbst können auch mit Superabsorberteilchen versehen sein.

Die flüssigkeitsabsorbierende Materiallage kann lose in der zweiten Hülle angeordnet sein, oder aber mit der Hülle verbunden sein. Die Verbindung kann durch Kleben, Verschweißen und/oder durch Druckausübung ausgeführt sein, sie kann flächig oder aber punktuell bzw. linienförmig ausgeführt sein.

Als Klebemittel kommen alle pharmazeutisch akzeptablen Klebemittel in Frage. Insbesondere kommen Polysaccharide enthaltende Kleber (wie z.B. Stärkekleber), Eiweiße enthaltende Kleber oder Acrylatkleber, wie sie auch als Gewebekleber Verwendung finden, in Frage. Weiterhin können, wie die Erfinder festgestellt werden, z.B. Gele oder W/O- bzw. O/W-Emulsionen verwendet werden, um die beiden Matten adhäsiv (d. h. als Klebemittel im obigen Sinne) miteinander zu verbinden. Die Verschweißung kann z.B. durch Ausübung von Wärme und/oder Ultraschall ggf. in Verbindung mit Druck ausgeführt werden.

Bevorzugt wird jedoch eine Ausführung des Wundpflegeartikels, bei der sowohl die zweite flüssigkeitsabsorbierende Materiallage als auch die erste, innere Hülle sowie der in der inneren Hülle befindliche erste flüssigkeitsabsorbierende Körper frei beweglich sind.

In dieser Ausgestaltung weist der Wundpflegeartikel zwei verschiedene Seiten mit unterschiedlich hoher Saugleistung auf. Das Pflegepersonal kann dabei je nach Exsudationsgrad entscheiden, welche Seite des Wundpflegeartikels auf die Wunde aufgelegt werden soll. Bei stark exsudierenden Wunden wird die Seite, an welcher der erste flüssigkeitsabsorbierende Körper mit Superabsorber-Teilchen angeordnet ist, bevorzugt auf die Wunde aufgelegt, während bei schwach exsudierenden Wunden die Seite, an welcher die vorgenannte Material-Lage, beispielsweise Schaumstoff oder aber eine Matte aufweisend Natriumcyrboxymethylcellulose angeordnet ist, auf die Wunde aufgelegt wird. Sollte in letzterem Fall die Exsudation der Wunde im Laufe der Behandlung zunehmen, tritt Exsudat auf der der Wunde abgewandten Seite aus der cellulosehaltigen Matte oder aus dem Schaumstoff aus und wird dort zuverlässig von dem saugstarken ersten flüssigkeitsabsorbierenden Körper mit Superabsorber-Teilchen aufgenommen und gebunden.

Ein solchermaßen aufgebauter Wundpflegeartikel weist neben ihrer hohen Aufnahmekapazität für Exsudate die Eigenschaft auf, dass sie
a) nekrotische Partikel, Pathogene und insbesondere keime aufnimmt und zurückhält,
b) Exsudate aufnimmt und zurückhält, Schleime jedoch nicht aufnimmt, und
c) Aufgrund des relativ hohen Dampfdrucks eine hohe atmosphärische Feuchtigkeit erzeugt, die zur Generierung eines wundheilungsfördernden Feuchtklimas beiträgt.

Die Verwendung von Schaumstoff bzw. einer Hydrofasermatte trägt dem Wundrandschutz bei. Außer der Vermeidung von Mazeration wird auch Polsterwirkung des Schaumstoffes erzielt. Innerhalb des Wundraums kann eine gewünschte konstante Temperatur über einen längeren Zeitraum aufrechterhalten werden, weil der Wundpflegeartikel relativ dicker ist und eine bessere Thermoisolation gewährleistet.

Es kann jedoch überdies vorgesehen sein, dass auch die Material-Lage, also die zweite flüssigkeitsabsorbierende Matte, mit Superabsorber-Teilchen versehen ist, um ihre Flüssigkeitsaufnahmekapazität zu erhöhen.

Bei den erwähnten Alginaten handelt es sich um Polysaccharide aus 1,4 verknüpfter α-L-Guluronsäure und β-D-Mannuronsäure. Alginate bilden homopolymere Bereiche, in denen Mannuronsäure oder Guluronsäure als Block vorliegt. Diese Blöcke werden als GG- oder MM-Blöcke bezeichnet. Im Bereich der GG- und MM-Blöcke kommt es zu einer Art Faltstruktur, die bei der Gelierung eine wesentliche Rolle spielt. Insbesondere die GG-Blöcke bilden eine regelmäßige Zickzack-Struktur aus.

In Form des faserigen Calcium- oder Calcium-Natrium-Alginats werden sie bekanntlich als Wundkompressen oder als Wundtamponade verwendet. Alginate haben eine große Absorptionskapazität und bilden durch die Aufnahme von Wundsekret und Mikroorganismen unter starker Quellung ein visköses, formstabiles Gel, das die Wunde feucht hält (Flüssigkeitsaufnahme bis zum 20-fachen des Eigengewichtes). Außerdem wirken Alginate aufgrund der Freisetzung von Calciumionen hämostatisch.
Der erste flüssigkeitsabsorbierende Körper kann auch von einer alginathaltigen Matte unterlegt sein, die ggf. ebenfalls innerhalb der inneren Hülle angeordnet ist. Vorzugsweise ist in dieser Ausführungsform die alginathaltige Matte der parallel angeordneten zweiten Materiallage abgewandt. Soll die Aufnahmekapazität der Alginatmatte unzureichend sein, nimmt der matteartige flüssigkeitsabsorbierende Körper das überschüssige Wundexsudat auf.

Ebenfalls kann mit der zweiten flüssigkeitsabsorbierenden Materiallage eine alginathaltige Matte flächig verbunden sein, die ggf. zwischen der zweiten flüssigkeitsabsorbierenden Materiallage (Matte) und der Hüllenwand der äußeren, zweiten Hülle liegt.

Das Vorhandensein der alginathaltigen Matte auf der dem ersten Körper abgewandten Seite kann durch das Fachpersonal als vorteilhaft angesehen werden, da es mehrere Auswahlmöglichkeiten gibt, einen Absorptionskörper von vorbestimmten Aufsaugkapazität für eine bestimmte Wundart und eingeschätzte Exsudatmenge anzuwenden. In dem Fall ist vorteilhaft, dass die alginathaltige Matte eine kleinere Fläche als die der zweiten, flüssigkeitsabsorbierenden Materiallage hat, weil es auf den wirksamen Wundrandschutz geachtet werden muss.

Die flüssigkeitsabsorbierende Materiallage kann gelocht sein. Dabei kann es sich bevorzugt um relativ große Löcher ggf. unterschiedlicher Geometrie handeln. Dabei können runde Löcher mit einem Durchmesser von 2 mm bis 15 mm vorgesehen sein. Ferner kann die gelochte Materiallage auf einer weiteren, vorzugsweise lochfreien flüssigkeitsabsorbierenden Matte, bevorzugt aus Natriumcyrboxymethylcellulose oder Schaumstoff aufliegen.

Die Grobpartikeln des Wundexsudats können an den Löchern haften. Das übrige dünnflüssige Wundexsudat gelangt über die offenzellige, hydrophile Schaumstruktur in ihre Tiefe. Es kann auch vorteilhaft sein, dass die der Hüllenwand zugewandte, gelochte Schaummatte größere Poren als die der anderen, aufliegenden Matte aufweist. Darüber hinaus ist auch denkbar, als Wundauflage einen sogenannten Integralschaum einzusetzen, bei dem sich um eine einzige Schaumschicht handelt, deren Porendichte sich kontinuierlich, in dem Fall von der wundnahen Seite beginnend in Richtung Wundabdeckung hin verkleinert.

Vorzugsweise bestehen die innere und/oder die äußere Hülle aus hydrophobem Material, beispielsweise aus Polypropylen oder aus einem hydrophob ausgerüsteten Naturmaterial, wie Baumwolle.

Die hydrophoben Eigenschaften der Hülle (insbesondere der äußeren Hülle) verhindern das Verkleben mit der Wundoberfläche und tragen dazu bei, dass die Wundexsudat-Partikeln schneller ins Innere der Hülle gelangen können.

Die beiden Hüllen können aus anderen Kunststoff-Folien, insbesondere einer Polyurethan- oder Polyethylenfolie oder aus künstlicher Spinnenseiden Folie hergestellt sein.

Das Material der inneren und/oder der äußeren Hülle kann derart strukturiert sein, dass die Hülle eine raue Innenfläche und eine glatte Außenfläche aufweist. Vorzugsweise ist die raue Innenfläche der Hülle durch trichterförmige Perforationen gebildet, die sich jeweils in Richtung Innenfläche verjüngen und in eine freie Öffnungskante ("Auskragung") auslaufen. Diese raue Innenfläche wirkt den Verschiebungen des Inhaltes der Hülle entgegen, so dass auf eine Fixierung mit Klebepunkten verzichtet werden kann. Dementsprechend kann die glatte Außenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden.

Vorzugsweise ist die äußere Hülle aus solchem "dreidimensionalen", dagegen die innere aus einem "zweidimensionalen", d. h. beidseitig ebenen Material gefertigt. Als Adjektiv "ebene" wird hier ein Aufbau des Hüllenmaterials verstanden, bei dem die Perforationen und dazwischen liegenden Materialabschnitte keine Erhebungen aufweisen. Um die Reibung zwischen den beiden Hüllen noch zu erhöhen, kann das Material der inneren Hülle rauh sein. Dabei können die Perforationen an beiden Hüllen unterschiedlich sein. Insbesondere können die Perforationen der äußeren Hülle größer als die der inneren sein, damit das dickflüssige Wundexsudat bzw. kleinere Zelltrümmer ins Innere der äußeren Hülle gelangen können. Durch die innere Hülle kommen dagegen dünnflüssige Wundexsudate hindurch, die letztendlich von der ersten Matte aufgenommen werden können.

Allerdings ist eine Ausführung der inneren und äußeren Hülle mit beiden glatten oder rauen Oberflächen auch möglich.

Der Wundpflegeartikel gemäß Erfindung kann lose auf die Wunde oder Körperhöhle gelegt und mit einem Sekundärverband oder einer Abdeckfolie ganzflächig am Körper des Patienten fixiert werden. Dementsprechend kann der Wundpflegeartikel Teil eines Okklusiv- oder Semiokklusivsystems oder auch Teil eines Kompressions- oder Unterdrucksystems sein.

Der Wundpflegeartikel kann mit eigenen Fixierungsmitteln ausgestattet sein, indem beispielsweise die Hüllenwände der äußeren Hülle an beiden ihren Flachseiten jeweils mit einem peripheren Klebestreifen versehen sind. Der doppelte Klebestreifen ermöglicht die Fixierung des Wundpflegeartikels mit einer durch das Fachpersonal ausgewählter Flachseite am Körper des Patienten. Es versteht sich, dass als Klebstoff medizinisch unbedenkliche, insbesondere bereits zugelassene Substanzen, beispielsweise auf Basis von Polysachariden in Frage kommen. Um die Handhabung des mit peripheren Klebestreifens versehenen Wundpflegeartikel zu erleichtern, ist es vorgesehen, die beiden gegenüberliegenden Klebestreifen jeweils mit einem abziehbaren, beispielsweise silikonartigen Schutzstreifen auszustatten.

Die Materiallage des beschriebenen Wundpflegeartikels kann wenigstens eine mittige, fensterartige Öffnung aufweisen, in die eine Einlage aus Alginat, insbesondere Calciumalginat oder auch ein Säckchen mit granulat- oder faserförmigen Superabsorber-Teilchen hineinpasst.

Wegen der Gefahr der Mazeration dürfen Matten aus bestimmten Alginaten nicht über den Wundrand hinaus reichen. Dem Erfordernis kommt eine so gestaltete Umrahmung des Alginat entgegen, die sowohl aus Natriumcyrboxymethylcellulose als auch Schaumstoff bestehen kann. Die Alginateinlage kann einen direkten Kontakt mit der jeweiligen Wunde haben, indem an der Hüllenwand der äußeren Hülle ein Fenster vorgesehen ist.

Den Komponenten des Wundpflegeartikels, insbesondere der Matte können Metallionen, insbesondere Zink-, Mangan- Calcium- oder Silberionen zur Unterstützung der lokalen Wundheilungsprozessc zugegeben sein. Weiterhin können Honig und dessen Derivate, Enzyme, Saponine, insbesondere pflanzlicher Herkunft (diese sind beispielsweise im Spitzwegerich *Plantago lanceolata* enthalten) oder andere desinfizierende Substanzen sowie Vitamine, wie Vitamin A, B9, B12. C und E, eingesetzt werden. Bevorzugt werden dabei insbesondere solche Komponenten, die radikalfangende bzw. antioxidative Eigenschaften haben.

Die Schaumstoff-Matte kann offen- oder geschlossenporig, hydrophil oder hydrophob sein. Wird ein geschlossenporiger Schaumstoff eingesetzt, so wird empfohlen, an dem Schaumstoff durchgehende Öffnungen bzw. Löcher einzubringen.

In Kombination mit einem Schaumverband in einer gemeinsamen zusätzlichen, äußeren Hülle werden weitere Vorteile erzielbar. So werden zwei Formen von Wundruhe erreicht:
Bei starker Exsudation wird die Seite des Wundpflegeartikels, die in der äußeren Hülle flächenhaft die erste flüssigkeitsabsorbierende Matte zeigt, zur Wunde appliziert, und über den erwünschten starken Saugeffekt entstresst der Wundpflegeartikel die Wundumgebung, indem er ihr das unerwünschte und mit schädlichen Boten- und Inhaltsstoffen versehene pathologische Exsudat entzieht. Interstitielle und interzellulare, aber auch zelluläre und auch vaskuläre Räume erreichen die Nähe einer physiologischen Bewässerung, so dass Perfusion, arterieller Zustrom, venöser Abtransport und transmembranöse Diffusion optimiert werden und nicht durch lange Diffusionswege, durch pathologische wässrige Lösungen und reaktive biorelevante Enzyme behindert werden. Denn Zellwachstum benötigt den arteriellen Anstrom von Proteinen, Sauerstoff, wie auch den Einschuss von Gefäßen, Nerven und Funktionsträgern der Immunabwehr.

Im Gegensatz zu diesen Erkenntnissen sieht die bisherige Praxis der Therapie vor, Verbände vergleichsweise niedriger Saugkraft und mangelhafter Retention zu applizieren. Oft kommen Schaumverbände zum Einsatz, die auf Druck das wenige aufgenommene Wasser mitsamt seinen wundheilungsschädlichen Inhaltsstoffen wieder freigeben. Der Fokus gerät damit auf die Wundoberfläche und beschäftigt sich mit der Bekämpfung von Folgeerscheinungen und der vordergründigen Beherrschung der kosmetischen Aspekte.

Es kann also empfehlenswert sein, als vollflächige primäre Kontaktschicht die SchaumstoffMatte zu nutzen, sofern die Vorzüge der mit Superabsorber-Teilchen durchsetzten Matte zusätzliche Verwendung finden. Dieses kann dadurch geschehen, dass der Schaum Wundkontakt hat und die genannte Matte ihm rückseitig direkt aufliegt, um zum einen die Angleichung an eine sehr inhomogene Wundbodenmorphologie über einen Schaum sicherzustellen, der als Durchfluss- und Kontaktkörper die Wundflüssigkeiten direkt in die Matte leitet. Auch die Erfüllung dieser Funktion über Alginate, Kohleverbände oder Baumwoll- und Vliesstoffe ist denkbar.

Bei schwächerer Exsudation kann die Schaumseite zur Wunde appliziert werden. Hier erzielt der Wundpflegeartikel einen mittelbaren Faktor der Wundruhe, indem er seine Saugkraft als Rücktrockner des Schaums nutzt. Hierzu ist es vonnöten, dass der Schaum annähernd durchfeuchtet, so dass z.B. durch Kapillarwirkungen der Durchstrom durch den Schaum gelingt und als sanfterer Einstrom in die flüssigkeitsabsorbierende Matte entsteht. Hier trocknet der Wundpflegeartikel den Schaum zurück und bildet ein sekundäres Reservoir, das die Kapazität des Schaums um die Kapazität der Matte erhöht, ohne dass dieser nennenswerten Kontakt zur Wunde selbst hat.

Der Wundpflegeartikel gemäß Erfindung kann als Mehrphasen-V-erband, insbesondere Mehrphasen-Primärverband bezeichnet werden, der eine verbesserte Kontrolle von austretender Wundflüssigkeit erlaubt. Das Wundexsudat strömt in den Schaum ein und wird dort "zwischengespeichert". Der Überschuß an Wundexsudat wird dann durch den inneren Absorptionskörper, der aus der cellulosehaltigen Matte und der inneren Hülle besteht, aufgesogen.

Werden in den Wundverbänden Schäume verwandt, so neigen diese dazu, sich an ihren Rändern aufzurollen. Die Verwendung eines durch die Matte und innere Hülle gebildeten "inneren" Absorptionskörpers rückseitig ruft hier einen erwünschten mechanischen Gegendruck hervor und trägt so zur Beibehaltung der vollflächigen Kontaktfläche mit der Wunde bei.

Das erfindungsgemäße Prinzip des einzelnen Wundpflegeartikels ermöglicht es, eine optische, vereinfachte Kontrolle des austretenden Wundexsudats bei Verwendung von derartigen, mit Superabsorber-Teilchen durchsetzten Absorptionskörpem durchzuführen, vorausgesetzt, dass der Absorptionskörper in Form von entsprechenden, an die Wunde angepassten Typengrößen eingesetzt wird.

Von großem Vorteil ist, dass das aufgesogene Wundexsudat eine definierte Position in dem Wundpflegeartikel einnehmen und behalten kann, wodurch die wundbenachbarte Umgebungshaut vom Wundexsudat nicht angegriffen wird.

Der Wundpflegeartikel gemäß Erfindung kann mit einer dehnbaren Abdeckfolie versehen sein, die die jeweilige maximale Volumenzunahme des Wundpflegeartikels während des Absorptionsvorgangs kaum oder gar nicht hindert. Eine solche Abdeckfolie kann beispielsweise gekreppt und/oder plissiert und/oder falten- und/oder balgenartig gelegt sein. Hierzu wird auf die DE 102007019622 der Anmelderin der vorliegenden Anmeldung verwiesen, deren Offenbarungsgehalt hier vollumfänglich einbezogen sein soll. Insbesondere vorteilhaft kann sich eine gekreppte Abdeckfolie erweisen, da sie in allen Richtungen dehnbar ist. Soll die plissierte oder gekreppte Abdeckfolie über eine Peripherie des Wundpflegeartikels hinausragen, so empfehlt sich, ihre über der Wundpflegeartikel ragende Fläche thermisch zu glätten.

Der Wundpflegeartikel gemäß Erfindung kann einer Wundfüllungsfunktion gerecht werden, wenn die alginat- oder schaumartige Materiallage mithilfe eines Stanzwerkzeugs oder eines anderen thermischen und/oder mechanischen Verfahrens eine Schneckenform erfährt. Die besagte alginat- oder schaumartige Materiallage kann auch aus mehreren konzentrisch angeordneten, runden oder rechteckigen Rahmen bestehen.

Vorzugsweise sind die Rahmen miteinander über aus demselben Material bestehende und versetzt angeordnete Brücken verbunden, so dass die zu mehreren Rahmen gestanzte Materiallage beim Entfalten nicht zerfällt.

In beiden Fällen kann die gestanzte Materiallage beim Auflegen auf die Wunde aus der wundnahen Hüllenwand der zweiten Hülle wenigstens teilweise als "Docht" hervortreten, wenn an der Hüllenwand entsprechende, sich kreuzende Einschnitte eingearbeitet sind. Dabei sollen die Einschnitte derart ausgeführt sein, dass sich der ins Wundinnere entfaltende Docht durch die entstandenen recht- bzw. dreieckigen Hüllenwand-Abschnitte nicht gebremst wird. Dementsprechend wird es vorgeschlagen, die Einschnitte diagonal bzw. radial an der wundnahen Hüllenwand anzuordnen. Beim Einsatz übt der Docht einen Druck auf die Hüllenwand aus, wodurch die Hüllenwand-Abschnitte von ihrer Flachlage abweichen und den Durchtritt des Dochtes ermöglichen. Der oberhalb des Dochtes liegende erste Körper unterstützt den Absorptionsprozess.

Wie bereits erwähnt, kann wenigstens eine der Hüllenwände der zweiten Hülle mit einer entsprechenden Hüllenwand der ersten Hülle eine gemeinsame Hüllenwand bilden. Diese Ausführung ist besonders vorteilhaft bei einer wesentlich kleineren Hüllenwand der ersten Hülle, welche über eine umlaufende Naht mit der Hüllenwand der zweiten Hülle verbunden ist. So können in Draufsicht auf die Hüllenwand der zweiten Hülle zwei umlaufende, konzentrisch angeordnete Nähte vorhanden sein. Beim aufgequollenen Wundpflegeartikel zieht sich die innere Naht zusammen und eine Verbreiterung der ersten Körpers verhindert, insbesondere dann, wenn der erste saugfähige Körper eine starke Ausbauchung erfährt. Eine solche Ausführung des Wundpflegeartikels ist insbesondere für tiefe Wunden geeignet.

Das beschriebene Konstruktionsprinzip umfasst auch zwei volle Hüllen, von denen die aus zwei Hüllenwänden bestehende erste Hülle über eine umlaufende Naht mit der aus zwei Hüllenwänden bestehenden zweiten Hülle verbunden ist, wobei die erste Hülle kleiner als die zweite ist.

Bevorzugt ist außerdem vorgesehen, dass der Wundpflegeartikel außerdem mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um einen BLIS (bacteriocin like inhibitory substance) oder um beschichtete magnetische Partikel handeln.

Bei dem nutritiv wirkenden Wirkstoff und/oder Wirkstoffkomplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Bei dem Proteasen hemmend wirkenden Wirkstoff und/oder Wirkstoffkomplex kann es sich um mindestens ein Wirkelement ausgewählt aus der Gruppe enthaltend Proteasehemmer, superabsorbierende Polymere, Chelatoren für zweiwertige Kationen, Kollagen, beschichtete magnetische Partikel, Säuren, Puffer, nicht pathogene säureproduzierende Mikroorganismen, Probiotika und/oder Symbiotika handeln.

Weitere Zusammenhänge und Hintergründe zu den nutritiven, einen desinfizierenden bzw. dekontaminierenden und/oder Proteasen hemmend wirkenden Wirkstoffen und/oder Wirkstoffkomplexen sind in der DE102007030931 der Anmelderin der vorliegenden Anmeldung beschrieben, auf deren Inhalt hier vollumfänglich Bezug genommen wird. In der DE102007030931 sind auch weitere nutritive, desinfizierende bzw. dekontaminierende und/oder Proteasen hemmend wirkende Wirkstoffen und/oder Wirkstoffkomplexe beschrieben, die ebenfalls als in dieser Anmeldung offenbart gelten sollen.

Weiterhin kann der erfindungsgemäße Wundpflegeartikel auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart.

Aus erstgenannter ist eine Vorrichtung zur Wundbehandlung unter Einsatz von Unterdruck bekannt, aufweisend ein gasdichtes Wundabdeckungselement , das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einem flächenhaften, die Wundsekrete aufnehmenden Wundpflegeartikel unterlegt ist, dessen Volumen im Laufe des Absorptionsprozesses zunimmt, so dass die absorbierten Wundsekrete innerhalb des Wundpflegeartikels und damit unterhalb des Wundabdeckungselementes bis zur Entfernung des Wundpflegeartikels aus dem Körper des Patienten verbleiben, der Wundpflegeartikel wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich der Wundpflegeartikel in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend:
ein Wundabdeckungselement zur Anbringung an Haut- und Schleimhautoberfläche,
wenigstens eine Anschlussstelle, die mit dem Wundraum in Kontakt steht und über welche die im Wundraum befindlichen Stoffe evakuiert werden können, wobei dieser superabsorbierende Polymere aufweist, wobei die absorbierten Wundsekrete an Polymere gebunden im Wundraum bis zur Entfernung aus dem Wundraum verbleiben,
wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens einen innerhalb des Raumes angeordneten, die Wundsekrete aufsaugenden Wundpflegeartikel, der wenigstens eine Lage eines mit Superabsorbentien angereicherten Textilabschnittes aufweist, die mit einer flüssigkeitsdurchlässigen Hülle umgeben ist, wobei die absorbierten Wundsekrete innerhalb des Wundpflegeartikel und damit unterhalb des Wundabdeckungselementes bis zur Entfernung des Wundpflegeartikel aus dem Körper des Patienten verbleiben, und wobei das Wundabdeckungselement eine gasdicht verschliessbare Behandlungsöffnung aufweist, durch die der Wundpflegeartikel in den Raum einlegbar und aus dem Raum entnehmbar ist.

Der Erfindungsgemäße Wundpflegartikel kann überdies eine an anatomische Gegebenheiten angepasste Form aufweisen. Hierzu kann er z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepaßten Verbandes.

Der erfindungsgemäße Wundpflegartikel kann außerdem so ausgebildet sein, dass er sich zur Umlage um eine chirurgisch angelegte Leitung eignet. Hierzu kann der Wundpflegeartikel z. B. wenigstens einen Schlitz aufweisen, der es ermöglicht, den Verband am Körper eines Patienten um eine Leitung (z.B. eine Drainageleitung oder einen Kathether) umzulegen,
wobei dem Wundpflegeartikel ein zweiter, ebenfalls flächenhafter Wundpflegeartikel zugeordnet ist, der von dem ersten Wundpflegeartikel in einem Abstand liegt,
wobei der Abstand durch einen Verbindungsstreifen oder -steg überbrückt ist. Ein so lcher Wundpflegartikel ist z.B. aus der DE202006005966 der Anmelderin der vorliegenden Erfindung bekannt.

Ebenso ist in diesem Zusammenhang bevorzugt vorgesehen, dass der Wundpflegeartikel mindestens ein Agenz aufweist, das die Blutung oder die Blutungsneigung einschränken kann.

Bei besagtem Agens kann es sich um mindestens einen chemisch und/oder physiologisch wirkenden Wirkstoff bzw. Wirkstoffkomplex oder um mindestens ein physikalisch wirkendes Wirkelement handeln. Ein solcher Wundpflegeartikel ist z.B. aus der DE102007036755 der Anmelderin der vorliegenden Anmeldung bekannt.

Hierzu kann der Wundpflegeartikel beispielsweise
- als im wesentlichen flacher Materialabschnitt aufweisend Absorptionsmaterial, der aus einem aufsaugenden Vlies mit darin verteilten superabsorbierenden Polymeren sowie mindestens einem chemisch und/oder physiologisch wirkende Wirkstoff bzw. Wirkstoffkomplex ausgebildet sein,
- als oder in Kombination mit einem Druck- oder Kompressionsverband,
- als eine Kombination aus einer primären, nicht oder nur unwesentlich absorbierenden Wundauflage, die mindestens einen chemisch und/oder physiologisch wirkenden Wirkstoff bzw. Wirkstoffkomplex aufweist, und einer peripher von dieser primären Wundauflage angeordneten sekundären Wundauflage, die superabsorbierende Polymere enthält, wobei ggf. zwischen beiden eine Diffusionsbarriere angeordnet ist,
- in Form eines Verbandpäckchens, aufweisend einen primäre Wundauflage mit mindestens einem chemisch und/oder physiologisch wirkenden Wirkstoff bzw. Wirkstoffkomplex sowie einem an dem Wundpflegeartikel angeordneten Wickelabschnitt, der zumindest abschnittsweise superabsorbierende Polymere aufweist, und/oder
- als Materialabschnitt mit einer Längserstreckung aufweisend Absorptionsmaterial, wobei der Materialabschnitt elastisch verformbare Eigenschaften aufweist, und wobei der Materialabschnitt superabsorbierende Polymere sowie ggf. mindestens einen chemisch und/oder physiologisch wirkende Wirkstoff bzw. Wirkstoffkomplex aufweist.

Bevorzugt handelt es sich bei dem chemisch und/oder physiologisch wirkenden Wirkstoff bzw. Wirkstoffkomplex um mindestens einen Stoff bzw. eine Zusammensetzung, die Blutstillende Eigenschaften aufweist. Diese Stoffe sind unter dem Oberbegriff "Hämostatika" bekannt.

Bevorzugt handelt es sich bei dem chemisch und/oder physiologisch wirkenden Wirkstoff bzw. Wirkstoffkomplex um mindestens einen Stoff bzw. eine Zusammensetzung, die Blutstillende Eigenschaften aufweist. Diese Stoffe sind unter dem Oberbegriff "Hämostatika" bekannt. Unter dem Begriff "chemisch und/oder physiologisch wirkenden Wirkstoff bzw. Wirkstoffkomplex" sollen in diesem Zusammenhang also solche Wirkstoffe bzw. Wirkstoffkomplexe verstanden werden, die die Blutung oder die Blutungsneigung einzuschränken imstande sind, ohne dass dabei physikalische Kräfte angewendet werden müssen. Der Wirkungsweg ist hier eine chemische und/oder physiologische Interaktion mit dem Wundmilieu.

Bei dem physikalisch wirkenden Wirkelement handelt es sich z.B. um eine Abbindung, ein Druckpolster, einen Druckverband oder einen Kompressionsverband. Unter dem Begriff "physikalisch wirkendes Wirkelement" soll in diesem Zusammenhang also ein Wirkelement verstanden werden, das auf physikalischem Wege, d.h. durch die Ausübung von Druck, Zug, Kälte und dergleichen, die Blutung oder die Blutungsneigung einzuschränken imstande ist.

Unter dem Begriff "Wirkstoffkomplex" soll im Folgenden nicht nur ein Komplex im chemischen Sinne verstanden werden, sondern insbesondere eine Zusammensetzung synergistisch eine Wirkung hervorrufender Wirkstoffe.

Die besagten Wirkstoffe bzw. Wirkstoffkomplexe können als Instantgranulat oder -pulver vorliegen.

Einige beispielhafte Konfigurationen des erfindungsgemäßen Wundpflegeartikels sind in,der folgenden Tabelle noch einmal aufgeführt:

| | **Erster saugfähiger Körper** | **(Zweite) saugfähige Matte** | **Hüllen-Konfiguration** | **Abbilung** |
|---|---|---|---|---|
| 1 | Airlaid-Matte mit Superabsorber, kreisförmig | PUR-Schaumstoff oder CMC mit oder ohne Superabsorber, rechteckig | Hülle in Hülle Periphere Verbindung | (Fig. 9) |
| 2 | Airlaid-Matte in Schnipsel-form mit Superabsorber | PUR-Schaumstoff oder CMC mit oder ohne Superabsorber | Hülle in Hülle oder Hülle auf Hülle | Fig. 22 Fig. 17 |
| 3 | Airlaid-Matte mit Superabsorber | PUR-Schaumstoff mit Lochstanzungen | Hülle in Hülle | Fig. 2 |
| 4 | Airlaid-Matte mit Superabsorber | CMC mit Fenster, dort eine Alginateinlage | Hülle in Hülle | Fig. 12, Fig. 13 |
| 5 | Airlaid-Matte mit Superabsorber | PUR-Schaumstoff (ggf.. als Schnipsel) oder CMC | Hülle auf Hülle | Fig. 17 a |
| 6 | Airlaid- oder Alginat-Matte mit oder ohne Superabsorber | PUR-Schaumstoff oder CMC mit oder ohne Superabsorber | Verbandheft | Fig. 20, Fig. 21 |
| 7 | Airlaid- oder Alginat-Matte mit oder ohne Superabsorber | PUR-Schaumstoff gestanzt (spiral- oder kastenförmig) | Hülle in Hülle oder Hülle auf Hülle oder Verbandheft | Fig. 25, Fig. 26, Fig. 28 |
| 8 | Alginat-Matte mit oder ohne Superabsorber | PUR-Schaumstoff oder CMC in kaskadierter Form (pyramidenartig) | Hülle in Hülle | Fig. 27 b |

Es versteht sich, dass im Rahmen der vorliegenden Erfindung eine Vielzahl weiterer Kombinationen möglich sind.

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken. Die Ausführungsbeispiele sind anhand der Figuren näher erläutert. Die Figuren zeigen:
- Fig.1a: einen Wundpflegeartikel gemäß Erfindung in ihrer ersten Ausführungsform, vor dem Zusammenschweißen der Hüllenwände, in einer schematischen Explosionsdarstellung;
- Fig. 1b: den gebrauchsfertigen Wundpflegeartikel gemäß Fig. 1a, in einem Schnitt senkrecht zu einer Flachseite des Wundpflegeartikels;
- Fig. 2: eine zweite Ausführungsform des Wundpflegeartikels, mit Fixierungsmitteln, ebenfalls in einem Schnitt senkrecht zu einer Flachseite des Wundpflegeartikels;
- Fig. 3: eine dritte Ausführungsform des Wundpflegeartikels, mit einer strukturierten äußeren Hülle;
- Fig. 4: eine vierte Ausführungsform des Wundpflegeartikels, mit einer strukturierten äußeren Hülle und einer peripher an der inneren Hülle angebrachten SchaumstoffMatte;
- Fig. 5: eine fünfte Ausführungsform des Wundpflegeartikels, mit einer auf den Schaumstoff aufkaschierten Alginat-Lage;
- Fig. 6: eine sechste Ausführungsform des Wundpflegeartikels, mit doppelter Matte innerhalb der ersten, inneren Hülle;
- Fig. 7: der Wundpflegeartikel gemäß Fig. 1b in Draufsicht auf ihre Flachseite;
- Fig. 8: Teil des Wundpflegeartikels gemäß Fig. 1b in einer perspektivischen Ansicht;
- Fig. 9: eine siebte Ausführungsform des Wundpflegeartikels, in Draufsicht auf ihre Flachseite;
- Fig. 10: eine achtc Ausführungsform des Wundpflegeartikels, mit einer mittig angeordneten Alginat-Einlage, in Draufsicht auf ihre Flachseite;
- Fig. 11: einen Schnitt A-A gemäß Fig. 10;
- Fig. 12: Anordnung zweier Schaumstoff-Matten und der Alginat-Einlage in einer perspektivischen Explosionsdarstellung;
- Fig. 13: eine neunte Ausführungsform des Wundpflegeartikels, mit einer Alginat-Einlage und einem Fenster, ebenfalls in Draufsicht auf die Flachseite des Wundpflegeartikels;
- Fig. 14: Verwendung eines Wundpflegeartikelsgemäß Fig. 1 in einem Ungerdrucksystem, in einer schematischen Ansicht;
- Fig. 15: Verwendung eines Wundpflegeartikelsgemäß Fig. 1 in einem Okklusivsystem, ebenfalls in einer schematischen Ansicht;
- Fig. 16: eine elfte Ausführungsform des Wundpflegeartikels, in einer perspektivischen Ansicht;
- Fig. 17a: einen Schnitt B-B gemäß Fig. 16,
- Fig. 17b: den aufgequollenen Wundpflegeartikelgemäß Fig. 16, ebenfalls im Schnitt;
- Fig. 18: eine zwölfte Ausführungsform des Wundpflegeartikels, dargestellt wie im Schnitt gemäß. Fig. 17a;
- Fig.19: eine dreizehnte Ausführungsform des Wundpflegeartikels, ebenfalls dargestellt wie im Schnitt gemäß Fig. 17a;
- Fig. 20: eine vierzehnte Ausführungsform des Wundpflegeartikels, hier in aufgeklapptem Zustand, in Draufsicht auf ihre Flachseite;
- Fig. 21a: Der Wundpflegeartikel gemäß Fig. 20 während des Zusammenklappens; in einer perspektivischen Ansicht;
- Fig. 21b: den zusammengeklappten Wundpflegeartikelgemäß Fig. 21a, in einer schematischen Seitenansicht;
- Fig. 22: eine fünfzehnte Ausführungsform des Wundpflegeartikels, in einer schematischen Seitenansicht;
- Fig. 23: eine sechzehnte Ausführungsform des Wundpflegeartikels, mit Schnipseln in der inneren Hülle, in einer schematischen Seitenansicht;
- Fig. 24: eine siebzehnte Ausführungsform des Wundpflegeartikels, mit Schnipseln in einer zusätzlichen inneren Hülle, in einer schematischen Seitenansicht;
- Fig. 25a: eine achtzehnte Ausführungsform des Wundpflegeartikels, mit einer schneckenförmig gestanzten, alginathaltigen Material-Lage, in einer schematischen Seitenansicht;
- Fig. 25b: der Wundpflegeartikel gemäß Fig. 25a in einer Draufsicht auf ihre wundzugewandte Seite;
- Fig. 26: eine neunzehnte Ausführungsform des Wundpflegeartikels, mit einer aus mehreren konzentrisch angeordneten, rahmenartigen Materialabschnitten bestehenden Material-Lage, in einer Draufsicht auf ihre wundzugewandte Seite;
- Fig. 27a: den gebrauchsfertigen Wundpflegeartikel gemäß Fig. 26, in einer schematischen Seitenansicht,
- Fig. 27b: der Wundpflegeartikel gemäß Fig. 26 mit deformierter Material-Lage, in einer schematischen Seitenansicht; und
- Fig. 28: eine zwanzigste Ausführungsform des Wundpflegeartikels, ebenfalls in einer schematischen Seitenansicht.

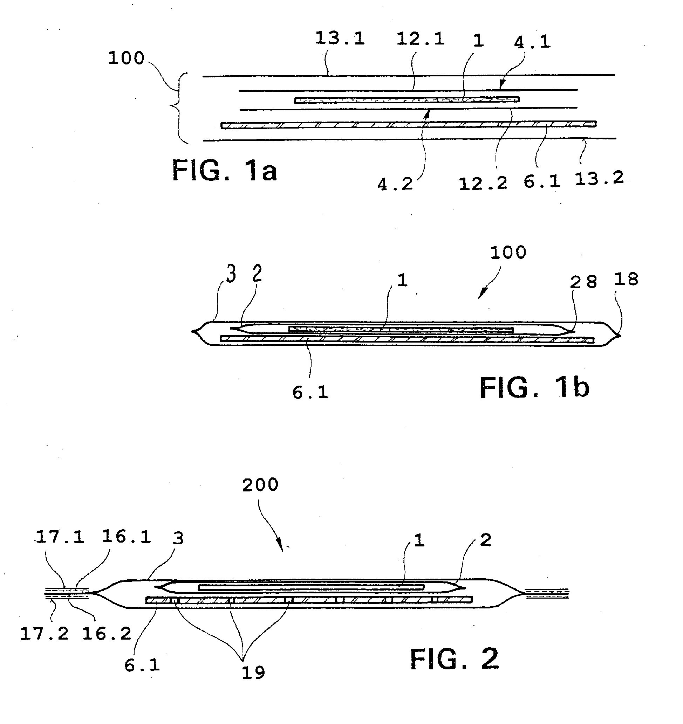

In Fig. 1a ist ein erster, mit Bezugszahl 100 bezeichneter Wundpflegeartikel dargestellt, bestehend aus zwei äußeren Hüllenwänden 13.1, 13.2, zwei inneren Hüllenwänden 12.1, 12.2 und einem zwischen den inneren Hüllenwänden 12.1, 12.2 positionierten ersten flüssigkeitsabsorbierenden Körper 1. Der besagte Körper 1 liegt in Form einer cellulosehaltigen, vliesartigcn Matte vor. Die Hüllenwand 12.1 ist mit ihrer ersten Flachseite 4.1 auf die äußere Hüllenwand 13.1 und mit ihrer zweiten Flachseite 4.2 auf eine schaumartige Materiallage 6.1 gerichtet, die zwischen der inneren Hüllenwand 12.2 und der äußeren Hüllenwand 13.2 liegt.

Wie es insbesondere die Figuren 1b, 7 und 8 zeigen, sind die den Körper 1 umgebenden Hüllenwände 12.1, 12.2 sowie die äußeren Hüllenwände 13.1, 13.2 jeweils miteinander über eine periphere Ultraachallnaht.28; 18 zu einer ersten, inneren und entsprechend einer zweiten, äußeren Hülle 2; 3 zusammengeschweißt, so dass der matteartige Körper 1 von der inneren Hülle 2 und entsprechend die innere Hülle 2 sowie die schaumartige Materiallage 6.1 von der äußeren Hülle 3 vollständig umgeben sind. Alle Hüllenwände 12.1, 12.2; 13.1, 13.2 des Wundpflegeartikels 100 sind flüssigkeitsdurchlässig. Als Material zur Herstellung der Hüllenwände 12.1, 12.2; 13.1, 13.2 wird eine perforierte Polyurethanfolie verwendet.

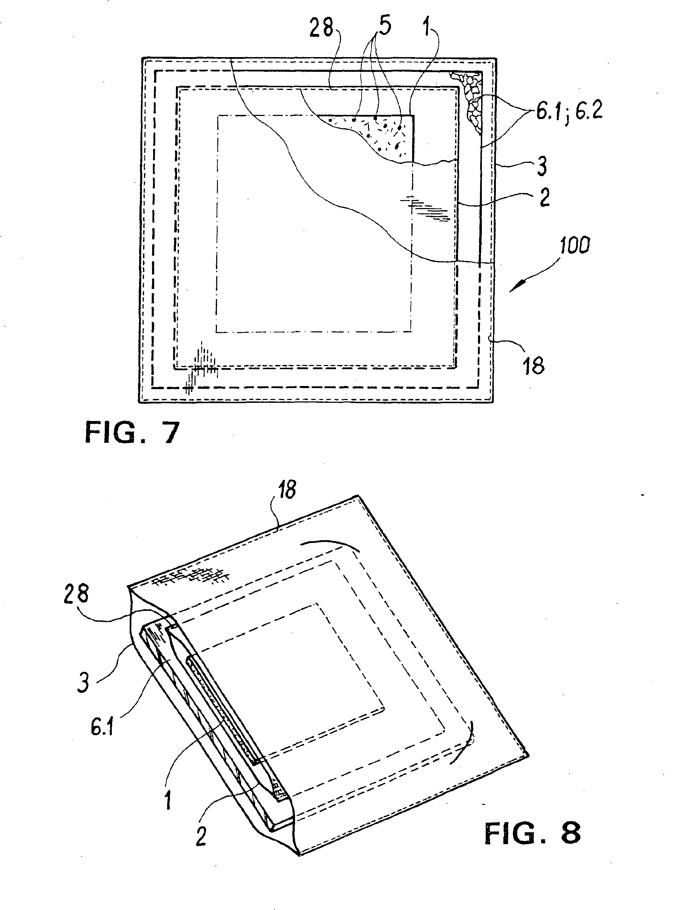

Zur Herstellung der Materiallage 6.1 kommt in vorliegendem Fall offenzelliger Polyurethan-Weichschaum von einer Dicke zwischen 2 mm und 3 mm in Frage. Insgesamt ist der entstandene neue Wundpflegeartikel 100 etwa 5 bis 6 mm dick. Die beiden Hüllen 2; 3 sowie die Materiallage 6.1 sind miteinander nicht verbunden. Auch der innerhalb der inneren Hülle 2 liegende mattenartige Körper 1 ist in der Hülle 2 frei beweglich angeordnet. Der mattenartige Körper 1 weist eine Menge von granulat- und pulverförmigen Superabsorber-Teilchen 5 auf (vgl. Fig. 7).

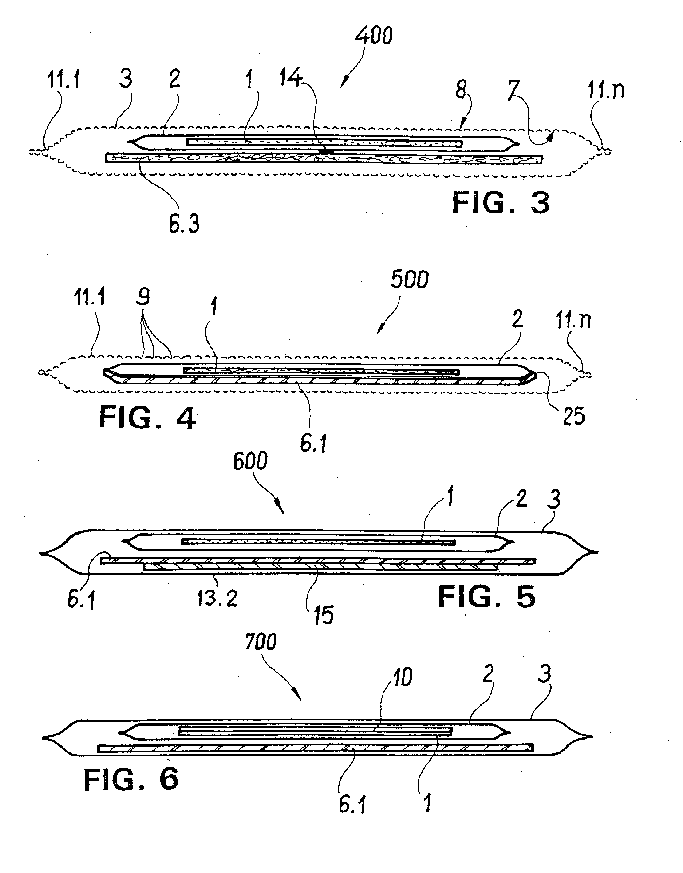

Optional kann die zweite flüssigkeitsabsorbierenden Matte an der inneren Hülle 2 über einen Klebstoffpunkt 14 fixiert sein. So zeigt die Fig. 3 ein Wundpflegeartikel 400, bei der über den mittig angeordneten Klebstoffpunkt 14 die Hüllenwand 12.2 der inneren Hülle 2 mit einer aus Natriumcarboxymethylcellulose gefertigten Materiallage 6.3 verbunden ist. Als Natriumcarboxymethylcellulose-Lage kommt beispielsweise das unter dem Markennamen HYDROFASER (Inhaber: E.R. Squibb & Sons, L.L.C., Princeton, N.J., USA) vertriebene Material zum Einsatz. Die aus Natriumcarboxymethylcellulose bestehende Materiallage 6.3 ist flächenmäßig größer als die innere Hülle 2. Die äußere Hülle 3 selbst ist aus einem hydrophoben Material, hier: Polyurethanfolie gefertigt, die trichterförmige Perforationen 9 und sich zwischen den Perforationen 9 erstreckende, gewölbte Materialabschnitte 11.1,....11.n aufweist, die einerseits eine raue Innenfläche 7 und andererseits eine glatte, wundschonende Außenfläche 8 ergeben.

Ein in Fig. 4 dargestellter Wundpflegeartikel 500 weist eine der Ausführungsform gemäß Fig. 3 identisch aufgebaute äußere Hülle 3 auf. Die äußere Hülle 3 umgibt ebenfalls die schaumartige Materiallage 6.1 und die den flachen Körper 1 beinhaltende innere Hülle 2, allerdings sind die innere Hülle 2 und die schaumartige Materiallage 6.1 miteinander über eine Schweißnaht 25 peripher verbunden.

Die Fig. 5 zeigt einen weiteren Wundpflegeartikel (fünfte Ausführungsform, Bezugszeichen 600), bei der sich um eine Weiterentwicklung des in Figuren 1a, 1b dargestellten ersten Wundpflegeartikels100 handelt. Zwischen Materiallage 6.1, im Weiteren Schaumstoff-Matte genannt, und der Hüllenwand 13.2 der äußeren Hülle 3 ist eine Alginat-Lage 15 angeordnet, die flächenmäßig etwas kleiner als die Schaumstoff-Matte 6.1 ist. Alternativ können die beiden Lagen 6.1 und 15 die gleiche Fläche haben. Die Alginat-Lage 15 ist in bekannter Weise auf die Schaumstoff-Matte 6.1 aufkaschiert.

Eine Weiterentwicklung des in Figuren 1a, 1b dargestellten ersten Wundpflegeartikels 100 ist auch der Fig. 6 zu entnehmen. Hierbei handelt sich um zwei innerhalb der inneren Hülle 2 flach liegende Matten, von denen die eine, der Materiallage 6.1 zugewandte, den flüssigkeitsabsorbierenden Körper 1 darstellt. Auf den Körper 1 ist eine weitere Matte 10 aus Calciumalginat aufkaschiert, wobei die beiden flächenmäßig gleich sind. Die alginathaltige Matte 10 ist der Schaumstoff-Matte 6.1 abgewandt.

Die Fig. 2 stellt ebenfalls eine Weiterentwicklung des in Figuren 1a, 1b dargestellten ersten Wundpflegeartikels 100 dar. Ein mit Bezugszeichen 200 bezeichneter Wundpflegeartikel ist mit Fixierungsmitteln versehen, die es erlauben, der Wundpflegeartikel am Körper des Patienten anzubringen. Zu diesem Zweck sind die beiden Hüllenwände 13.1, 13.2 der äußeren Hülle 3 an beiden ihren Außenflächen jeweils mit einem peripheren Klebestreifen 16.1, 16.2 sowie mit abziehbaren, Schutzstreifen 17.1, 17.2 aus Silikonpapier versehen. Die Schaumstoff-Matte 6.1 ist mit mehreren durchgehenden Öffnungen 19 (Löchern) von einem Durchmesser etwa 4 mm versehen. Vom Vorteil ist, dass die beidseitige Anordnung der Fixierungsmittel es ermöglicht, der Wundpflegeartikel mit ihrer beliebigen Flachseite am Körper des Patienten anzukleben.

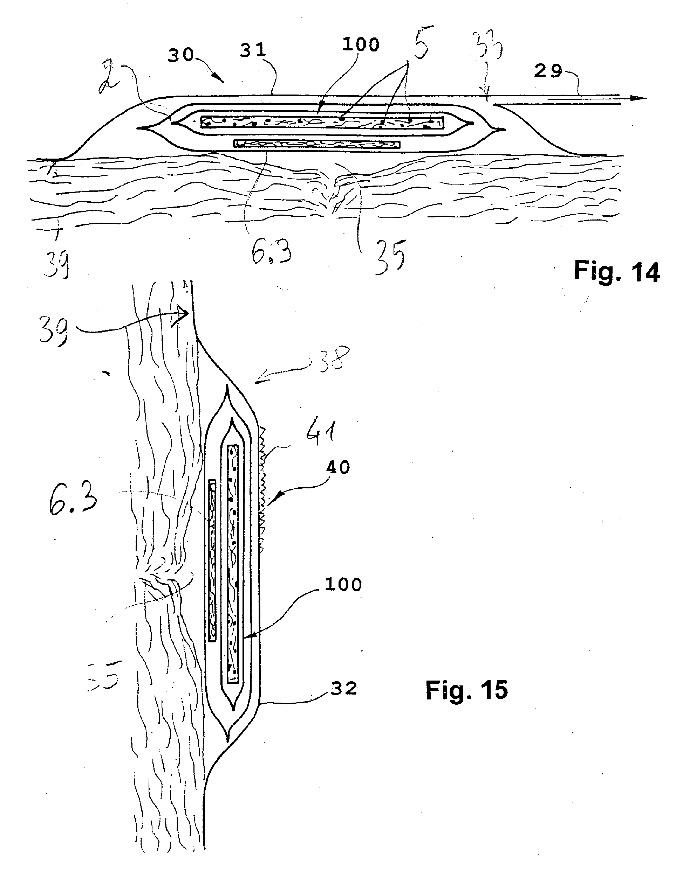

Die Figuren 14 und 15 zeigen schematisch eine Verwendung des Wundpflegeartikels 100 in einem Unterdrucksystem 30 und entsprechend in einem Okklusivsystem 40. Das Unterdrucksystem 30 beinhaltet eine flüssigkeits- und wasserdampfdichte Abdeckfolie 31 und eine sich über eine schematisch gezeigte Öffnung 33 an die Abdeckfolie 31 anschließende Vakuumleitung 29, die vorzugsweise etwa parallel zum Wundpflegeartikel 100 läuft. Der Wundpflegeartikel 100 beinhaltet wiederum eine alginathaltige, der Wunde 35 zugewandte Materiallage 6.3, welche flächenmäßig kleiner als der innerhalb der inneren Hülle 2 liegende, mattenartige Körper 1 ist. Der flüssigkeitsabsorbierende Körper 1 besteht aus vliesartig angeordneten Cellulosefasern und darin verteilten Superabsorber-Teilchen 5. Die Abdeckfolie 31 ist mit der Hüllenwand der äußeren Hülle 3 nicht verbunden.

Das Okklusivsystem 40 beinhaltet eine flüssigkeitsdichte, jedoch wasserdampfdurchlässige Abdeckfolie 32 und den besagten Wundpflegeartikel 100, die ebenfalls die alginathaltige, der Wunde 35 zugewandte Materiallage 6.3 aufweist, die flächenmäßig kleiner als der innerhalb der inneren Hülle 2 liegende Körper 1 ist.

Die beiden flexiblen Abeckfolien 31, 32 ragen über eine Peripherie 38 des Wundpflegeartikels hinaus und verfügen jeweils über eine periphere Klebefläche 39, damit problemlos an der Umgebungshaut der Wunde anbringbar sein können.

Die in Fig. 15 gezeigte Abdeckfolie 32 ist plissiert (gefaltet), so dass ihre Gesamtfläche gegenüber einer glatten Folie vergrößert ist. Dies ist vorteilhaft und ermöglicht eine starke Dehnung der Abdeckfolie 32 während des Absorptionsvorgangs, so dass eine erhebliche Volumenzunahme des Wundpflegeartikels im Wesentlichen keine nennenswerten, durch die Abdeckfolie ausgeübten Gegenkräfte hervorruft. Mit der Bezugszahl 41 sind schematisch Falten der Abdeckfolie 32 bezeichnet. In einem anderen, nicht dargestellten Ausführungsbeispiel ist die Abdeckfolie nicht plissiert, sondern gekreppt. Die kreppartige Abdeckfolie kann sich in allen Richtungen dehnen.

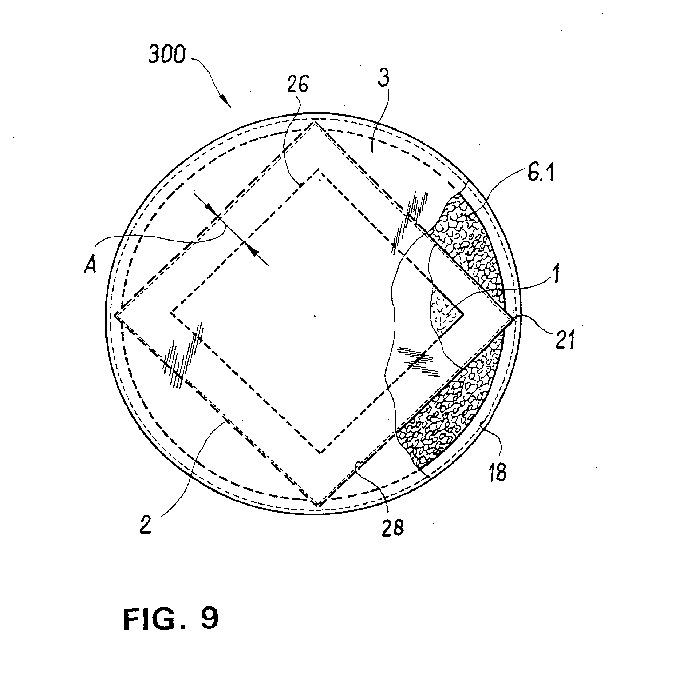

Ein runder Wundpflegeartikel 300 ist aus der Fig. 9 ersichtlich. Rund ist jedoch nur die äußere Hülle 3 und die Schaumstoff-Matte 6.1, dagegen sind die innere Hülle 2 sowie der mattenartige Körper 1 in Draufsicht auf die Flachseite des Wundpflegeartikels 300 quadratisch, wobei die Ausmaße der inneren Hülle 2 so an das Innere der äußeren Hülle 3 angepasst sind, dass die innere Hülle 2 mit ihren allen vier Ecken 21 nahezu bis zur Ultraschallnaht 18 reicht. Auf diese Weise werden die unerwünschten Verschiebungen der inneren Hülle innerhalb der äußeren Hülle mit einfachen mitteln ausgeschlossen, ohne dass es zum (u.U. aus medizinischen Gründen nicht erwünschten) Einsatz von Klebstoff kommt. Eine solche Konfiguration ist Herstellungstechnisch sehr leicht zu verwirklichen. Wie der Fig. 9 zu entnehmen ist, ist der mattenartige Körper 1 flächenmäßig kleiner als innere Hülle 2, so dass sich während der Aufsaugung voll innerhalb der Hülle 2 entfalten kann. Ein mit Bezugszeichen A bezeichneter Abstand zwischen der Ultraschallnaht 18 der inneren Hülle 2 und einer Außenkante 26 des Körpers 1 kann am Umfang variabel sein bei Beibehaltung der angegebener Ausmaße des Körpers 1 und der inneren Hülle 2.

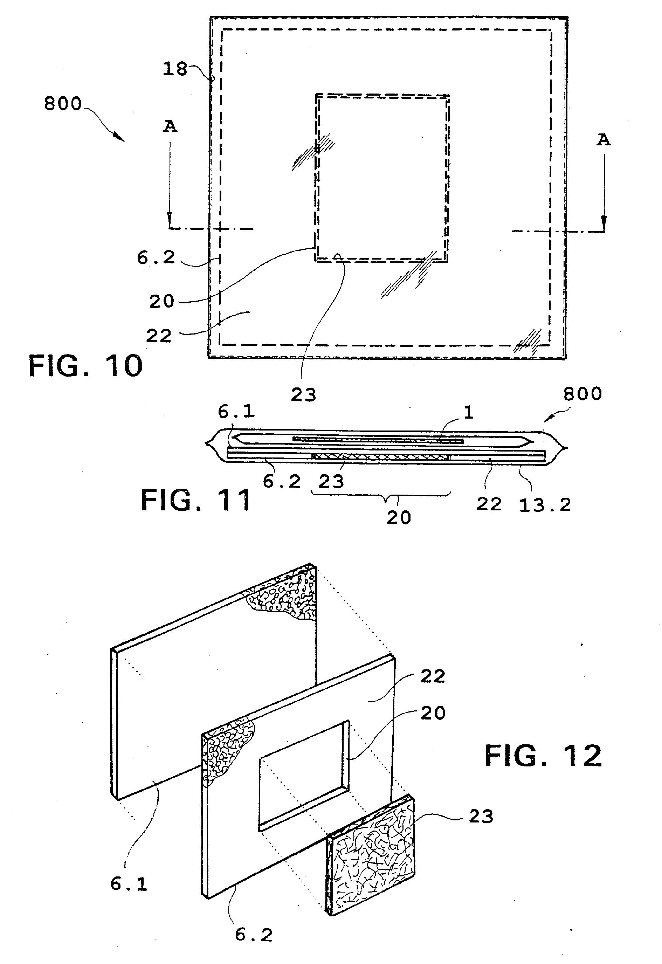

In Figuren 10 und 11 ist ein Wundpflegeartikel 800 gezeigt, die sich aus der inneren Hülle 2 mit darin untergebrachtem vliesartigen Körper 1, der äußeren Hülle 3 und zwei aufeinander liegenden Schaumstoff-Matten 6.1, 6.2 zusammensetzt, wobei an der der Hüllenwand 13.2 zugewandten Schaumstoff-Matte 6.2 eine rechteckige, mittig angeordnete Öffnung 20 ausgestanzt ist, so dass eine Umrahmung 22 (vgl. insbesondere Fig. 12) für eine in die Öffnung 20 platzierte, alginathaltige Einlage 23 entstanden ist.

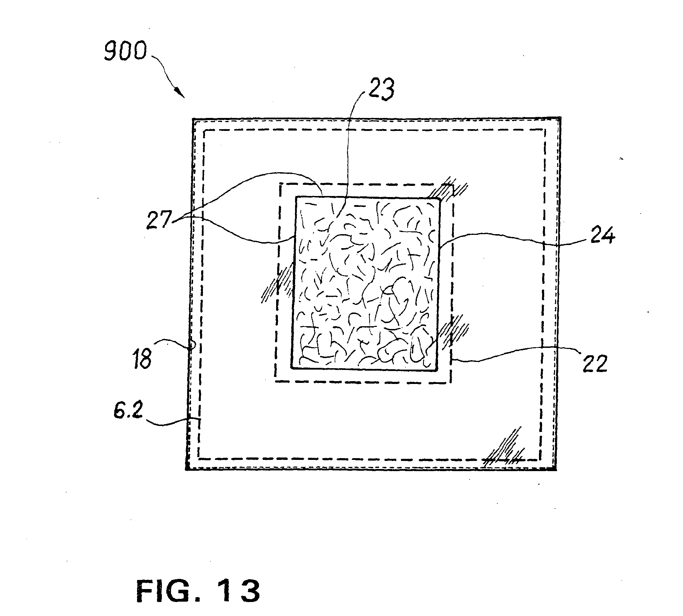

In Fig. 13 ist ein ähnlicher Wundpflegeartikel 900 dargestellt, bei der an der Hüllenwand 13.2 der äußeren Hülle 3 ein Fenster 24 ausgestanzt ist, dessen Kanten 27 die alginathaltige Einlage 23 etwas überlappen. So wird der Einlage 23 ein ausreichender Halt innerhalb der Öffnung 22 gewährleistet. Die alginathaltige Einlage 23 kann mit der jeweiligen Wunde zum direkten Kontakt kommen. Optional kann in die alginathaltige Einlage 23 eine superabsorbierende Substanz in Pulver-, Granulat- oder Faserform eingearbeitet werden.

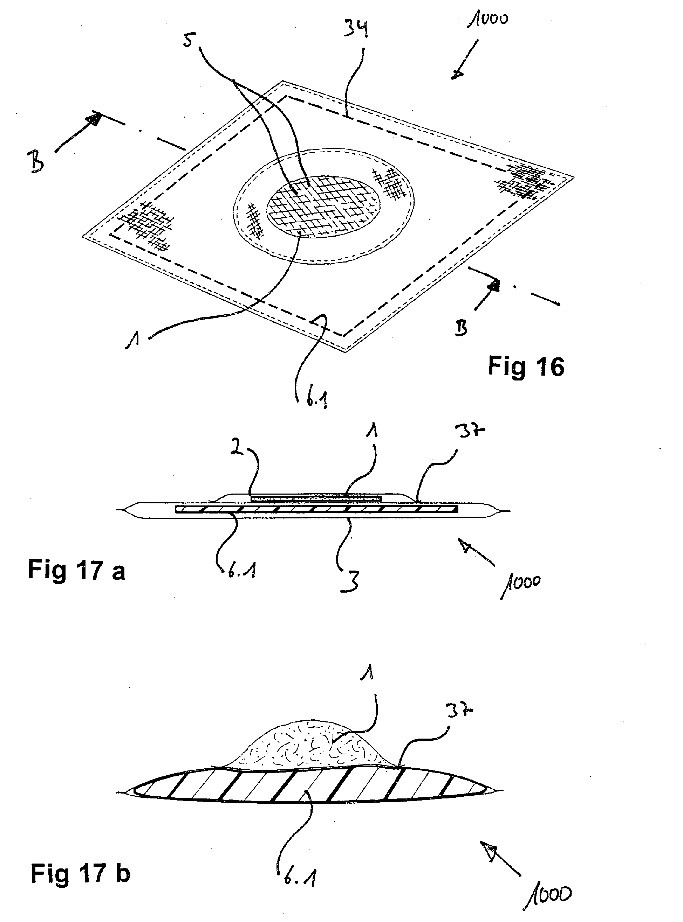

Die Figuren 16, 17a und 17b zeigen ein Wundpflegeartikel 1000, bei der mit der Hüllenwand 13.1 der zweite Hülle 3 eine wesentlich kleinere Hüllenwand 12.1 der ersten Hülle 2 über eine umlaufende Ultraschallnaht 37 verbunden ist. Auf eine zweite Hüllenwand 12.2 der ersten Hülle 2 wird verzichtet. Die kleinere, einen runden Umriss 34 aufweisende Hülle 2 ist mittig an der zweiten Hülle 3 angeordnet. Innerhalb der zweiten Hülle 3 ist die schaumartige (Polyurethanschaum) Materiallage 6.1 und entsprechend innerhalb der ersten Hülle 2 eine cellulosehaltige Matte (Körper 1) beweglich angeordnet. Die größere, im vorliegenden Fall quadratische Hülle 3 hat Ausmaße 10 x 10 cm, dagegen die kleinere einen Durchmesser von etwa 6 cm, bemessen jeweils bis zur entsprechenden Ultraschallnaht. Bei diesen Abmessungen der beiden Hüllen 2; 3 ist die Materiallage 6.1 etwa 6,5 cm x 6,5 cm groß, dagegen hat der mattenartige Körper 1 einen Durchmesser von etwa 4 cm.

Der in Fig. 17b dargestellte Wundpflegeartikel 1000 nach einem Saugprozess zeigt eine mäßig aufgequollene Schaumstoff-Matte 6.1 und einen stark aufgequollenen, etwa halbkugeligen Körper 1. Diese Konfiguration des aufgequollenen Wundpflegeartikels mit einer Einseitig konvexen Oberfläche kann einer Oberflächenbeschaffenheit einer tiefen Wunde entsprechen, diese ausfüllen und so die Heilung beschleunigen. Hinzu kommt, dass durch die konvexe Oberfläche ein dauerhafter Kontakt zwischen absorbierenden Materialien und dem Wundboden gewährleistet ist, was eine dauerhafte Exsudataufnahme gewährleistet. Diese Vorteile gelten im Übrigen für alle Ausgestaltungen, die eine konvexe Oberfläche und/oder eine Wundfüllung aufweisen bzw. ermöglichen.

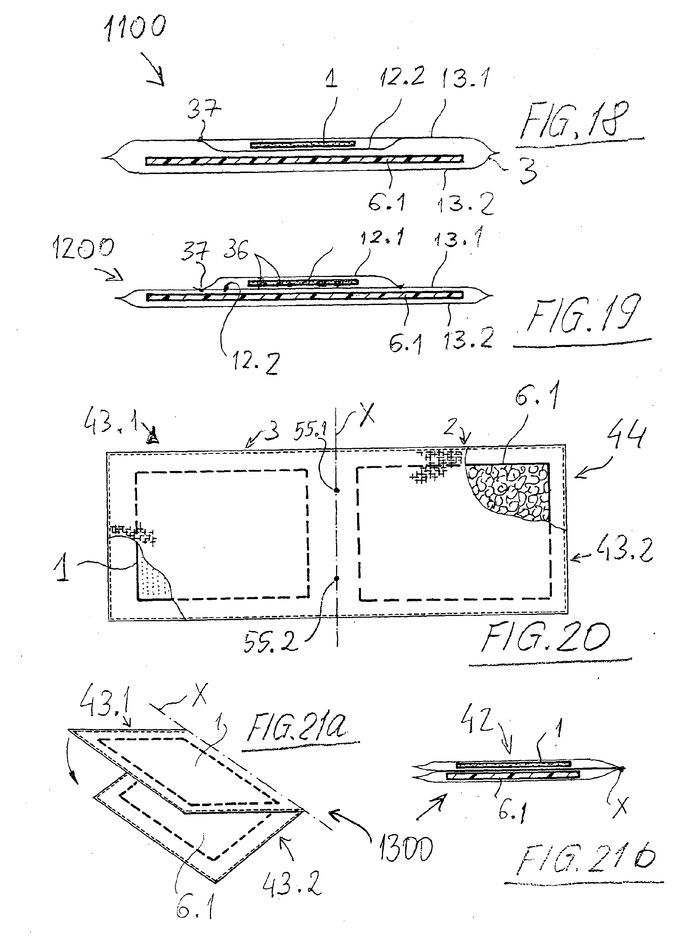

Anstelle des Körpers 1, der aus cellulosehaltiger Matte mit darin verteilten Superabsorber-Teilchen 5 gefertigt ist, können andere stark saugende Materialien, wie eine Alginat-Matte, eine Schüttung aus Schnipseln und/oder aus Superabsorber-Teilchen eingesetzt werden. So zeigt die Fig. 19 ein Wundpflegeartikel 1200, deren Aufbau dem des Wundpflegeartikels 1000 sehr ähnlich ist, wobei die kleinere Hülle 2 eine Schüttung aus Schnipseln 36 beinhaltet. Die Schnipsel 36 von einer durchschnittlichen Größe 3 mm bis 5 mm sind aus einer celluloseartigen, mehrschichtigen Airlaid-Matte, die mit Superabsorber-Teilchen angereichert ist, maschinell geschnitten. Die Versuche haben gezeigt, dass eine der ganzen Matte gewichtsgleiche Schüttung aus Schnipseln 36 die gleiche Flüssigkeitsmenge etwa 15% schneller aufnehmen kann. Ursache hierfür ist vermutlich die vergrößerte Oberfläche der Schnipsel-Ausführungsform im Vergleich zu einer gewichtsgleichen Airlaid-Matte.

Ein besonderer Vorteil ist, dass die Schnipsel 36 auch aus einem Abfallprodukt bei der Mattenherstellung bestehen können.

Vorteilhaft ist bei dieser Ausführung ist auch, dass die Schnipsel anders als eine flächige Matte sich beim Aufquellen dreidimensional ausdehnen und eine konvexe Oberfläche ausbilden, ähnlich wie dies eine Schüttung tut, und so z.B. Wundhöhlen ausfüllen können.
Diese Eigenschaft entfaltet eine die Heilung beschleunigende Wirkung. Durch die Ausfüllung wird ein dauerhafter Kontakt zwischen absorbierenden Materialien und dem Wundboden gewährleistet, was eine dauerhafte Exsudataufnahme sicherstellt. Diese Vorteile gelten im Übrigen für alle Ausgestaltungen, die eine konvexe Oberfläche und/oder eine Wundfüllung aufweisen bzw. ermöglichen.

In einem weiteren Ausführungsbeispiel (nicht dargestellt), das sich ebenfalls auf der Wundpflegeartikel 1000 bezieht, besitzt die kleinere Hülle 2 ihre beide Hüllenwände 12.1, 12.2. Daraus resultiert, dass die vollständige kleinere Hülle 2 auf die zweite, größere Hülle 3 aufgelegt und mit dieser peripher verbunden ist.

Die Fig. 18 zeigt ein Wundpflegeartikel 1100, bei der die erste, kleinere Hülle 2 mit ihrer der Materiallage 6.1 zugewandten Hüllenwand 12.2 an der der Materiallage 6.1 abgewandten

Hüllenwand 13.1 der zweiten Hülle 3 vermittels der peripheren Ultraschallnaht 37 angebracht ist. Die Funktion der zweiten, nicht vorhandenen Hüllenwand 12.1 der ersten Hülle 2 übernimmt die Hüllenwand 13.1 der zweiten, größeren Hülle 3. Sonst besteht der Wundpflegeartikel 1100 aus denselben Teilen, die bei der Beschreibung des Wundpflegeartikels 1000 (Figuren 16, 17a und 17b) erwähnt worden sind.

Bei einer in den Figuren 20, 21a und 21b dargestellten Wundpflegeartikel 1300 handelt es sich um ein Verbandheft 42 (vgl. Fig. 21a), das durch Zusammenklappen zweier Flügel 43.1, 43.2 einer gemeinsamen Doppelhülle 44 entlang einer Faltlinie X entstanden ist. Die Fig. 20 zeigt, dass die aufgeklappte Doppelhülle 44 aus der ersten Hülle 2 und der zweiten Hülle 3 besteht, wobei die erste in einem Materialstück in die zweite Hülle 3 übergeht. Die Flügel 43.1, 43.2 setzen sich jeweils aus der Hülle 2 oder 3 und entsprechenden saugfähigen Materialien zusammen. In vorliegendem Ausführungsbeispiel beinhaltet die erste Hülle 2 ein stark aufsaugendes Textilmaterial, in dem Fall den bereits erwähnten mattenartigen Körper 1 und die zweite Hülle 3 die ebenfalls beschriebene schaumartige Materiallage 6.1. An der Faltlinie X liegen zwei Punktnähte 55.1, 55.2, die die beiden flächenmäßig gleichen Hüllen 2; 3 derart voneinander trennen, dass die innerhalb der Doppelhülle 44 befindlichen saugfähigen Materialien nicht über die Faltlinie verschoben werden können. Die Faltlinie X kann durch eine nicht gezeigte, durchgehende Naht ersetzt werden; sie kann jedoch auch perforiert sein und ein Trennen beider Hälften durch Reissen ermöglichen.

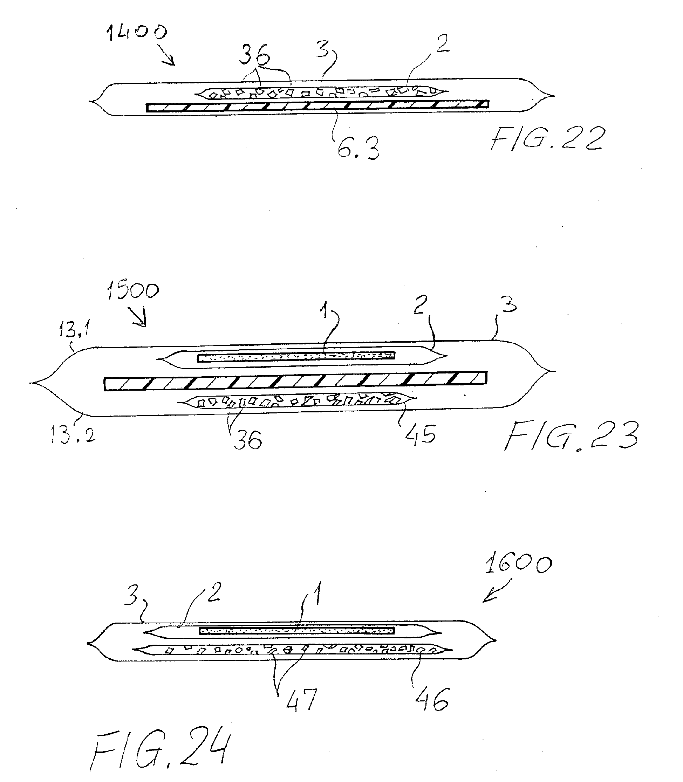

Bei einem in Fig. 22 dargestellten Wundpflegeartikel 1400 handelt es sich um eine "Hülle-in-Hülle"-Konfiguration, die beispielsweise auch die Fig.1b zeigt. Der Unterschied zwischen den beiden Wundpflegeartikeln 100 und 1400 besteht darin, dass die erste, innere Hülle 2 des Wundpflegeartikels 1400 bereits beschriebene, aus der Airlaid-Matte geschnittene Schnipsel 36 beinhaltet und dass die außerhalb der inneren Hülle 2 liegende Materiallage 6.3 aus Hydrofasern, d. h. aus Natriumcarboxymethylcellulose besteht, wobei die Materiallage 6.3 flächenmäßig größer als die innere Hülle 2 ist.

Die Fig. 23 zeigt wiederum ein Wundpflegeartikel 1500, bestehend aus der schaumartigen Materiallage 6.1 in der zweiten, äußeren Hülle 3, dem flüsigkeitsabsorbierenden, mattenartigen Körper 1 in der ersten, inneren Hülle 2, und einer Schüttung aus Schnipseln 36, die in einer zusätzlichen, inneren, der Hülle 2 abgewandten Hülle 45 angeordnet sind. Die beiden inneren Hüllen 2; 45 liegen also jeweils zwischen der Materiallage 6.1 und einer Hüllenwand 13.1, 13.2 der äußeren Hülle 3.

Der Fig. 24 ist ein Wundpflegeartikel 1600 zu entnehmen, aufweisend eine in der inneren Hülle 2 untergebrachte Airlaid-Matte (Körper 1) und eine zusätzliche innere Hülle 46, in der sich eine Schüttung aus Alginat-Würfeln bzw. -schnipseln 47 befindet, wobei die beiden inneren Hüllen 2 und 46 von der äußeren Hülle 3 umgeben sind. Die Alginat-Würfeln bzw. - schnipseln 47 können durch Hydrofaser-Schnipsel (nicht dargestellt) ersetzt werden.

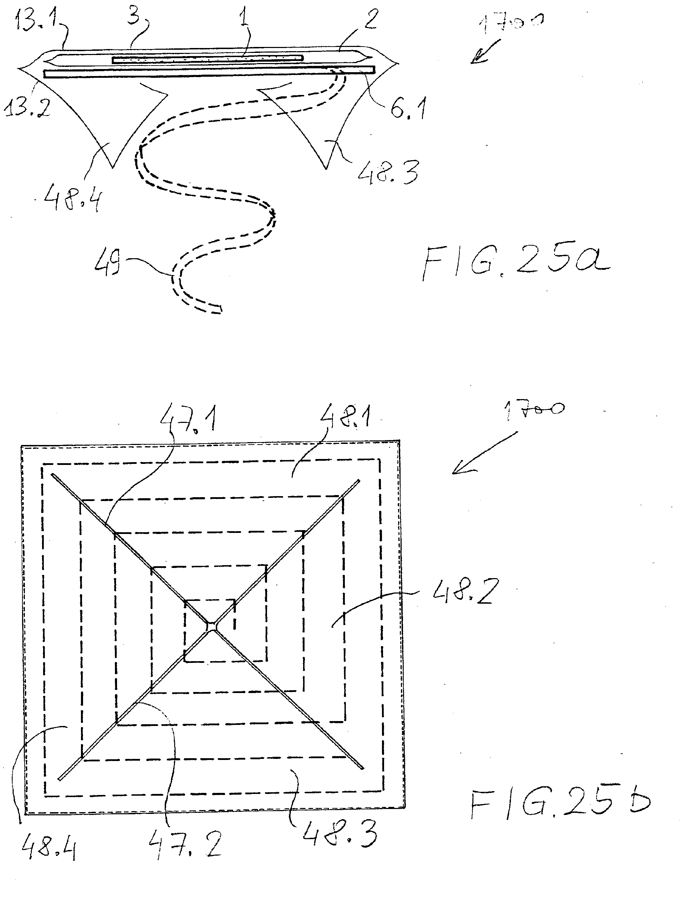

Die Figuren 25a und 25b zeigen einen materialmäßig dem Wundpflegeartikel 100 ähnlichen Wundpflegeartikel 1700. Die innere Hülle 2 umfasst den aus einer Airlaid-Matte gefertigten Körper 1, wobei die äußere Hülle 3 sowohl die innere Hülle 2 mit dem Körper 1 als auch die schaumartige Materiallage 6.1 (Polyurethan-Weichschaum), die jedoch schneckenförmig, wie aus der Fig. 25b ersichtlich, gestanzt ist. An der Hüllenwand 13.2 der äußeren Hülle 3 sind zwei sich kreuzende, diagonal verlaufende Einschnitte 47.1, 47.2 eingebracht, die diese Hüllenwand in vier dreieckige Hüllenwand-Abschnitte 48.1, 48.2, 48.3, 48.4 teilen, welche beim Einsatz (vgl. Fig. 25a) unter der auf die schneckenförmige Materiallage 6.1 wirkenden Schwerkraft leicht von ihrer ursprünglichen Flachlage abweichen und dadurch den Durchtritt wenigstens eines Teils der schneckenförmigen Materiallage 6.1 bzw. eines aus derselben entstandenen Dochtes 49 in Richtung Wunde ermöglichen. Der beschriebene Wundpflegeartikel 1700 erfüllt damit eine Dochtfunktion bei tiefen Wunden, wobei der oberhalb des Dochtes 49 liegende Körper 1 den Absorptionsprozess unterstützt. Hinzu kommt eine die Wunde ausfüllendc, die Heilung beschleunigende Wirkung. Durch die Ausfüllung wird ein dauerhafter Kontakt zwischen absorbierenden Materialien und dem Wundboden gewährleistet, was eine dauerhafte Exsudataufnahme sicherstellt. Diese Vorteile gelten im Übrigen für alle Ausgestaltungen, die eine konvexe Oberfläche und/oder eine Wundfüllung aufweisen bzw. ermöglichen.

Abstelle der diagonal verlaufenden Einschnitte 47.1, 47.2 können auch Stanzungen oder Perforationen bzw. Markierungen entlang der gezeichneten Linien vorgesehen sein. Die Linien können dem Pflegepersonal ein Aufschneiden ermöglichen, während durch Stanzungen oder Perforationen die besagten Einschnitte durch Reissen hergestellt werden können.

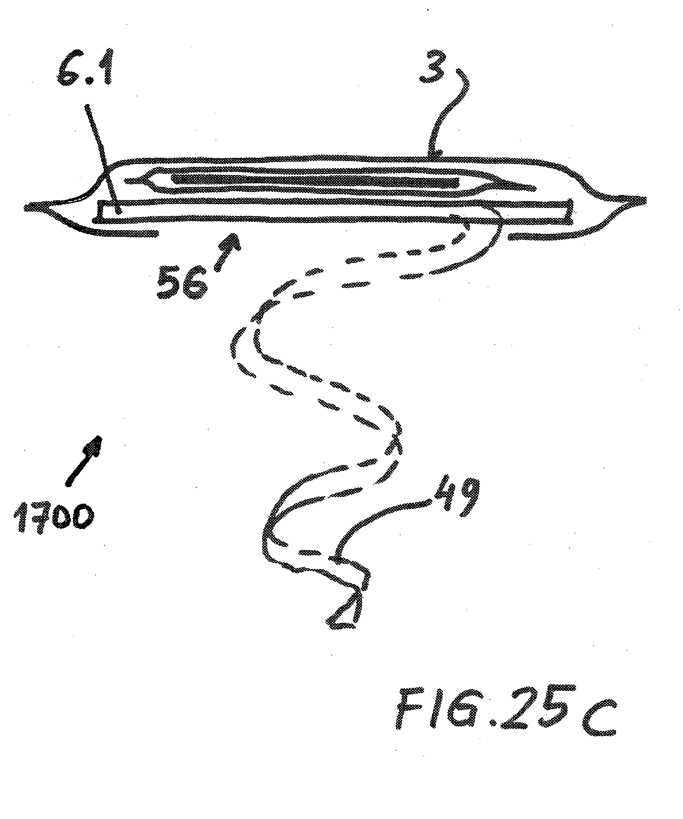

Die Figur 25c zeigt einen ähnlichen Wundpflegeartikel 1700, bei welchem auf der der Wunde zugewandten Seite der Hülle 3 ein Fenster 56 in der Hülle vorgesehen ist, wobei die Bemaßung des Fensters kleiner ausfällt als jene der in der äußeren Hülle vorgesehen Material-Lage 6.1. Letztere besteht aus einer schneckenförmig gestanzten Schaumstoffmatte und verbleibt im trockenen Zustand innerhalb der Hülle. Erst bei Kontakt mit Flüssigkeit, insbesondere mit Exsudat, tritt wenigstens ein Teils der schneckenförmigen Materiallage 6.1 bzw. eines aus derselben entstandenen Dochtes 49 aus der Hülle in Richtung Wunde heraus.

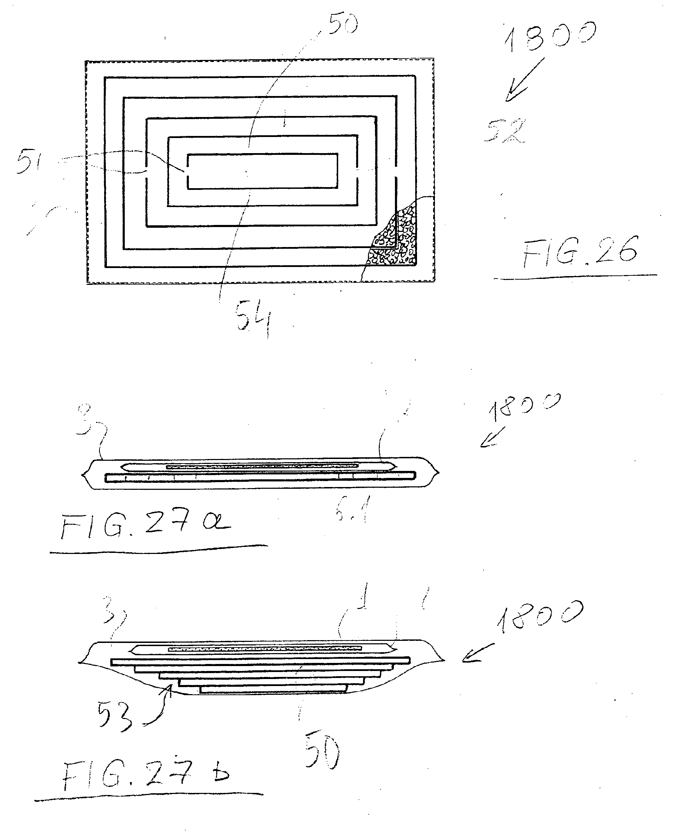

Die Figuren 26, 27a und 27b zeigen einen weiteren Wundpflegeartikel (Bezugszahl 1800) mit einer ebenfalls gestanzten, schaumartigen Materiallage 6.1, die jedoch zusammen mit dem innerhalb der ersten Hülle 2 liegenden mattenartigen Körper 1 vollständig von der äußeren Hülle 3 umgeben ist.

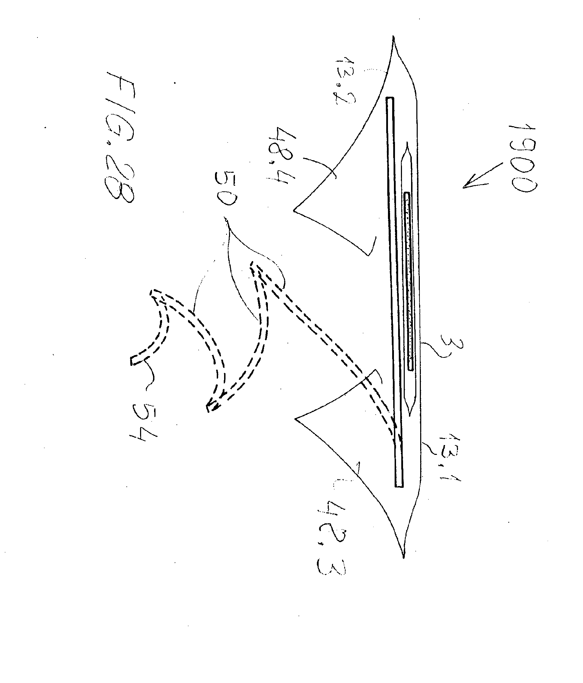

An der rechteckigen Materiallage 6.1 sind mehrere konzentrisch angeordnete, ebenfalls rechteckige Rahmen 50 mit einem Stanzwerkzeug ausgestanzt unter Belassung von die Rahmen 50 miteinander verbindenden Brücken 51, 52. Im Einsatzfall (vgl. Fig. 27b) deformiert sich die gestanzte Materiallage 6.1 zu einem deutlich dreidimensionalen Saugkörper 53, der sich auf die jeweilige Wunde, falls eine richtige Typengröße des Wundpflegeartikels gewählt worden ist, anpassen kann. Dadurch, dass an der Materiallage 6.1 Brücken 51, 52 angeordnet sind, zerfällt der Saugkörper 53 nicht. Voraussetzung für die richtige Funktion des beschriebenen "Zoom-Verbandes" ist eine ausreichend dehnbare Hülle. Sollen an Hüllenwand 13.2 der äußeren Hülle 3 ebenfalls vier deformierbare dreieckige Hüllenwand-Teile 48.1, 48.2, 48.3, 48.4 durch Einschnitte (wie in Figuren 25a, 25b) eingearbeitet sein, entsteht ein in Fig. 28 gezeigter Wundpflegeartikel 1900. Beim Auflegen auf die tiefe Wunde treten die miteinander über Brücken verbundenen Rahmen 50 durch die Hüllenwand 13.2 durch. Der kleinste Rahmen 50 ist über die Brücke 51 mit einem mittigen Materialabschnitt 54 verbunden, welcher am tiefsten liegt. Der Wundpflegeartikel 1900 ist eine Kombination aus den Wundpflegeartikeln 1700 und 1800.

Nicht dargestellt in Figs. 25 - 28, aber dem hier geschilderten Prinzip entsprechend, ist eine Ausführungsform, bei welcher die äußere Hülle bereits ein Fenster, also eine Aussparung aufweist, das den Blick auf die darunter angeordnete schaumartige, wie oben beschrieben gestanzte Materiallage freigibt, und dadurch den Durchtritt wenigstens eines Teils eines der schaumartigen Materiallage bzw. eines aus derselben entstandenen Dochtes in Richtung Wunde ermöglichen

Figur 27b stellt in einer alternativen Ausgestaltung einen weiteren Wundpflegeartikel (Bezugszahl 1800), bei dem mehrere Lagen einer nicht gestanzten schaumartigen Materiallage 6.1 unterschiedlicher Größe in kaskadierter bzw. pyramidenartiger Form übereinander angeordnet und zusammen mit dem innerhalb der ersten Hülle 2 liegenden mattenartigen Körper 1 vollständig von der äußeren Hülle 3 umgeben ist.

Diese Konfiguration kann einer Oberflächenbeschaffenheit einer tiefen Wunde entsprechen, diese ausfüllen und so die Heilung beschleunigen. Hinzu kommt, dass durch die Ausgestaltung ein dauerhafter Kontakt zwischen absorbierenden Materialien und dem Wundboden gewährleistet ist, was eine dauerhafte Exsudataufnahme gewährleistet

### Bezugszeichenliste:

- 1: flüssigkeitsabsorbierender Körper
- 2: Hülle
- 3: Hülle
- 4.1, 4.2: Flachseite
- 5: Superabsorber-Teilchen
- 6.1 bis 6.4: Material-Lage
- 7: Innenfläche
- 8: Außenfläche.
- 9: Perforationen
- 10: Matte
- 11 .n: Materialfeld
- 12.1, 12.2: Hüllenwand
- 13.1, 13.2: Hüllenwand
- 14: Klebstoffpunkt
- 15: Alginat-Lage
- 16.1, 16.2: Klebestreifen
- 17.1, 17.2: Schutzstreifen
- 18: Ultraschallnaht
- 19: Öffnung
- 20: Öffnung (v. 6.2)
- 21: Ecke
- 22: Umrahmung
- 23: Einlage
- 24: Fenster (v. 13.2)
- 25: Schweißnaht
- 26: Außenkante
- 27: Kante
- 28: Ultraschallnaht
- 29: Vakuumleitung
- 30: Unterdrucksystem
- 31: Abdeckfolie
- 32: Abdeckfolie
- 33: Öffnung
- 34: Umriss
- 35: Wunde
- 36: Schnipsel
- 37: Ultraschweißnaht
- 38: Peripherie
- 39: Klebefläche
- 40: Okklusivsystem
- 41: Falten
- 42: Verbandheft
- 43.1, 43.2: Flügel
- 44: Doppelhülle
- 45: zusätzliche Hülle
- 46: zusätzliche Hülle
- 47.1, 47.2: Einschnitt
- 48.1, 48.2: Hüllenwand-Abschnitt
- 48.3, 48.4: Hüllenwand-Abschnitt
- 49: Docht
- 50: Rahmen
- 51, 52: Brücke
- 53: dreidimensionaler Saugkörper
- 54: Materialabschnitt
- 55.1, 55.2: Punktnaht
- A: Abstand
- X: Faltlinie

- 100; 200; 300: Wundpflegeartikel
- 400; 500; 600: Wundpflegeartikel
- 700; 800; 900: Wundpflegeartikel
- 1000; 1100: Wundpflegeartikel
- 1200; 1300: Wundpflegeartikel
- 1400; 1500: Wundpflegeartikel
- 1600; 1700: Wundpflegeartikel
- 1800; 1900: Wundpflegeartikel

## Patentansprüche

1. Wundpflegeartikel (100; 200; 300; 400; 500; 600; 700; 800; 900; 1000; 1100; 1200; 1300; 1400; 1500; 1600; 1700; 1800; 1900) zur Extraktion und Kontrolle von Wundflüssigkeiten, aufweisend:
- wenigstens einen ersten flüssigkeitsabsorbierenden Körper (1; 10), der von einer flüssigkeitsdurchlässigen, ersten Hülle (2) umgeben ist,
- und eine flüssigkeitsdurchlässige, aus zwei Hüllenwänden (13.1, 13.2) bestehende, zweite Hülle (3),
**dadurch gekennzeichnet, dass**
die erste Hülle (2) an wenigstens einer ihrer Flachseiten (4.1, 4.2) von wenigstens einer flüssigkeitsabsorbierenden Materiallage (6.1, 6.2; 6.3) be- oder unterlegt ist, die zwischen der ersten Hülle (2) und einer der Hüllenwände (13.1, 13.2) der zweiten Hülle (3) angeordnet ist, und
wobei der erste flüssigkeitsabsorbierende Körper (1; 10) in Form einer vliesartigen Matte vorliegt und eine superabsorbierende Substanz aufweist,
und wobei die flüssigkeitsabsorbierende Materiallage Methylcellulose enthält.

2. Wundpflegeartikel nach Anspruch 1, **dadurch gekennzeichnet, dass** es sich bei der Methylcellulose um Natriumcarboxymethylcellulose handelt.

3. Wundpflegeartikel nach Anspruch 1 - 2, **dadurch gekennzeichnet, dass** die vliesartige Matte vom Typ Airlaid ist.

4. Wundpflegeartikel nach Anspruch 1, **dadurch gekennzeichnet, dass**
- die Hüllenwand (13.1) der zweiten Hülle (3) zugleich eine Hüllenwand (12.1; 12.2) der ersten Hülle (2) bildet,
- die Hüllenwand (12.1; 12.2) der ersten Hülle (2) über eine umlaufende Naht (37) mit der Hüllenwand (13.1) der zweiten Hülle (3) verbunden ist
- und dass die erste Hülle (2) flächenmäßig kleiner als die zweite Hülle (3) ist.

5. Wundpflegeartikel nach Anspruch 1, **dadurch gekennzeichnet, dass**
- die aus zwei Hüllenwänden (12.1, 12.2) bestehende erste Hülle (2) über eine umlaufende Naht (37) mit der Hüllenwand (13.1) der zweiten Hülle (3) verbunden ist,
- und dass und dass die erste Hülle (2) flächenmäßig kleiner als die zweite Hülle (3) ist.

6. Wundpflegeartikel nach einem der vorhergehenden Ansprüche, **dadurch gekennzeichnet, dass** die flüssigkeitsabsorbierende Materiallage (6.1, 6.2; 6.3) mindestens eine superabsorbierende Substanz (5) aufweist.

7. Wundpflegeartikel nach einem der vorhergehenden Ansprüche, **dadurch gekennzeichnet, dass** wenigstens die flüssigkeitsabsorbierende Materiallage (6.1, 6.2; 6.3) wenigstens abschnittsweise gelocht ist.

8. Wundpflegeartikel nach einem der vorhergehenden Ansprüche, **dadurch gekennzeichnet, dass** die erste und/oder die zweite Hülle (2; 3) eine raue Innenfläche (7) und eine glatte Außenfläche (8) aufweist.

9. Wundpflegeartikel nach einem der vorhergehenden Ansprüche, **dadurch gekennzeichnet, dass** diese Teil eines Unterdrucksystems (30) oder eines Kompressionssystems ist.

10. Wundpflegeartikel nach einem der vorhergehenden Ansprüche, **dadurch gekennzeichnet, dass** wenigstes eine der Hüllenwände (13.1, 13.2) der zweiten Hülle (3) durch eine Abdeckfolie (31; 32) abgedeckt ist.

11. Wundpflegeartikel nach Anspruch 10, **dadurch gekennzeichnet, dass** die Abdeckfolie (31; 32) adhäsiv ist.

12. Wundpflegeartikel nach einem der Ansprüche 10 bis 11, **dadurch gekennzeichnet, dass**
a) die Abdeckfolie (31; 32) flächenmäßig 5% bis 300% größer als die Hüllenwand (13.1, 13.2) der zweiten Hülle (3) ist, und/oder
b) die zweite Hülle (3) flächenmäßig 3% bis 300% größer als der flüssigkeitsabsorbierende Körper (1; 10) oder die Materiallage (6.1, 6.2; 6.3) ist.

13. Wundpflegeartikel nach einem der vorhergehenden Ansprüche, **dadurch gekennzeichnet, dass**
- an der Hüllenwand (13.1, 13.2) der zweiten Hülle (2) wenigstens ein Einschnitt (47.1, 47.2) eingebracht ist,
- und die schneckenförmige bzw. aus mehreren konzentrisch angeordneten Rahmen (50) bestehende Materiallage (6.1, 6.2; 6.3) beim Auflegen auf die Wunde aus der Hüllenwand (13.1, 13.2) wenigstens teilweise hervortritt.

14. Kit für die chronische Versorgung bzw. die Akut-, Notfall- oder Militärmedizinische bzw. die chronische Versorgung, aufweisend einen Wundpflegeartikel gemäß einem der vorherigen Ansprüche.

## Claims

1. Wound care article (100; 200; 300; 400; 500; 600; 700; 800; 900; 1000; 1100; 1200; 1300; 1400; 1500; 1600; 1700; 1800; 1900) for extraction and control of exudates, comprising:
- at least a first liquid absorbing body (1; 10), being surrounded by a first liquid permeable first shell (2),
- and a liquid permeable second shell (3) consisting of two shell walls (13.1, 13.2),
**characterised in that**,
the first shell (2) is covered or lined at least at one of its flat sides (4.1, 4.2) by at least one liquid absorbing material layer (6.1, 6.2; 6.3), which is arranged between the first shell (2) and one of the shell walls (13.1, 13.2) of the second shell (3), and
wherein the first fluid absorbing body (1, 10) is in the form of a fibre mat, comprising a super-absorbing substance,
and wherein the liquid absorbing material layer comprises methylcellulose.

2. Wound care article according to claim 1, **characterised in that** the methylcellulose is carboxymethylcellulose.

3. Wound care article according to claims 1 to 2, **characterised in that** the fibre mat is of Airlaid type.

4. Wound care article according to claim 1, **characterised in that**
- the shell wall (13.1) of the second shell (3) forms at the same time the shell wall (12.1, 12.2) of the first shell (2),
- the shell wall (12.1, 12.2) of the first shell (2) is connected by a circumferential seam (37) to the shell wall (13.1) of the second shell (3)
- and that the area of the first shell (2) is smaller than the second shell (3).

5. Wound care article according to claim 1, **characterised in that**
- The first shell (two) consists of two shell walls (12.1, 12.2) and is connected by a circumferential seam (37) with the shell wall (13.1) of the second shell (3),
- and that the surface area of the first shell (2) is smaller than the second shell (3).

6. Wound care article according to any one of the previous claims, **characterised in that** the liquid absorbing material layer (6.1, 6.2, 6.3) comprises at least one super-absorbing substance (5).

7. Wound care article according to any one of the previous claims, **characterised in that** the liquid absorbing material layer (6.1, 6.2, 6.3) comprises sections which are punched.

8. Wound care article according to any one of the previous claims, **characterised in that** the first and / or the second shell (2, 3) comprise a rough inner surface (7) and a smooth outer surface (8).

9. Wound care article according to any one of the previous claims, **characterised in that** the article is part of a vacuum suction system (30) or a compression system.

10. Wound care article according to any one of the previous claims, **characterised in that** at least one of the shell walls (13.1, 13.2) of the second shell (3) is covered by a sheeting (31, 32).

11. Wound care article according to claim 10, **characterised in that** the sheeting (31, 32) is adhesive.

12. Wound care article according to any one of the claims 10 to 11, **characterised in that**
a) the area of the sheeting (31, 32) is 5% to 300% larger than the shell walls (13.1, 13.2) of the second shell (3), and / or
b) the area of the second shell (3) is 3% to 300% larger than the liquid absorbing body (1, 10) or the material layer (6.1, 6.2, 6.3).

13. Wound care article according to any one of the previous claims, **characterised in that**
- at least one incision (47.1, 47.2) is formed at the shell wall (13.1, 13.2) of the second shell (2),
- and the material layer (6.1, 6.2, 6.3) is helical, respectively arranged in multiple concentrically frames (50) and emerges at least partially from the shell wall (13.1, 13.2) during application to the wound.

14. Kit for the chronic care respectively the acute-, emergency- or military medicinal respectively the chronic care, comprising a wound care article according to any one of the previous claims.

## Revendications

1. Article pour soin de plaies (100 ; 200 ; 300 ; 400 ; 500 ; 600 ; 700 ; 800 ; 900 ; 1000 ; 1100 ; 1200 ; 1300 ; 1400 ; 1500 ; 1600 ; 1700 ; 1800 ; 1900) pour l'extraction et le contrôle de liquides issus de plaies, présentant :
- au moins un premier corps absorbant du liquide (1 ; 10) qui est entouré d'une première enveloppe (2) perméable à du liquide,
- et une seconde enveloppe (3) perméable à du liquide et consistant en deux parois d'enveloppe (13.1, 13.2),
**caractérisé en ce que**
la première enveloppe (2) comporte au-dessus ou en-dessous d'au moins une de ses côtés plats (4.1, 4.2) au moins une couche de matériau absorbant du liquide (6.1, 6.2 ; 6.3) qui est disposée entre la première enveloppe (2) et une des parois d'enveloppe (13.1, 13.2) de la seconde enveloppe (3), et
le premier corps absorbant du liquide (1 ; 10) se présentant sous la forme d'une natte de type non-tissé et présentant une substance super-absorbante,
et la couche de matériau absorbant du liquide contenant de la méthylcellulose.

2. Article pour soin de plaies selon la revendication 1, **caractérisé en ce que** la méthylcellulose est de la carboxyméthylcellulose de sodium.

3. Article pour soin de plaies selon les revendications 1 - 2, **caractérisé en ce que** la natte de type non-tissé est de type appliqué par procédé aérodynamique par voie sèche (Airlaid).

4. Article pour soin de plaies selon la revendication 1, **caractérisé en ce que**
- la paroi d'enveloppe (13.1) de la seconde enveloppe (3) constitue en même temps une paroi d'enveloppe (12.1 ; 12.2) de la première enveloppe (2),
- la paroi d'enveloppe (12.1 ; 12.2) de la première enveloppe (2) est reliée par une suture périphérique (37) à la paroi d'enveloppe (13.1) de la seconde enveloppe (3)
- et que la première enveloppe (2), en termes de superficie, est plus petite que la seconde enveloppe (3).

5. Article pour soin de plaies selon la revendication 1, **caractérisé en ce que**
- la première enveloppe (2) consistant en deux parois d'enveloppe (12.1, 12.2) est reliée par une suture périphérique (37) à la paroi d'enveloppe (13.1) de la seconde enveloppe (3),
- et que la première enveloppe (2), en termes de superficie, est plus petite la seconde enveloppe (3).

6. Article pour soin de plaies selon une des revendications précédentes, **caractérisé en ce que** la couche de matériau absorbant du liquide (6.1, 6.2 ; 6.3) présente au moins une substance super-absorbante (5).

7. Article pour soin de plaies selon une des revendications précédentes, **caractérisé en ce qu'**au moins la couche de matériau absorbante de liquide (6.1, 6.2 ; 6.3) est perforée du moins par sections.

8. Article pour soin de plaies selon une des revendications précédentes, **caractérisé en ce que** la première et/ou la seconde enveloppe (2 ; 3) présente une surface intérieure rugueuse (7) et une surface extérieure lisse (8).

9. Article pour soin de plaies selon une des revendications précédentes, **caractérisé en ce qu'**il fait partie d'un système de dépression (30) ou d'un système de compression.

10. Article pour soin de plaies selon une des revendications précédentes, **caractérisé en ce qu'**au moins une des parois d'enveloppe (13.1, 13.2) de la seconde enveloppe (3) est recouverte par un film de recouvrement (31 ; 32).

11. Article pour soin de plaies selon la revendication 10, **caractérisé en ce que** le film de recouvrement (31 ; 32) est adhésif.

12. Article pour soin de plaies selon une des revendications 10 à 11, **caractérisé en ce que**
a) le film de recouvrement (31 ; 32), en termes de superficie, est plus grand de 5 % à 300 % que la paroi d'enveloppe (13.1, 13.2) de la seconde enveloppe (3), et/ou
b) la seconde enveloppe (3), en termes de superficie, est plus grande de 3 % à 300 % que le corps absorbant du liquide (1 ; 10) ou que la couche de matériau (6.1, 6.2 ; 6.3).

13. Article pour soin de plaies selon une des revendications précédentes, **caractérisé en ce que**
- au moins une incision (47.1, 47.2) est pratiquée sur la paroi d'enveloppe (13.1, 13.2) de la seconde enveloppe (2),
- et que la couche de matériau (6.1, 6.2 ; 6.3) de forme hélicoïdale ou composée de plusieurs cadres (50) disposés concentriquement ressort au moins partiellement de la paroi d'enveloppe (13.1, 13.2) lors de l'application sur la plaie.

14. Kit pour soins en cas de pathologies chroniques ou pour soins médicaux en cas de pathologies aiguës, d'urgence ou militaires ou chroniques, présentant un article pour soin de plaies selon une des revendications précédentes.
